# EUROPÄISCHE PATENTSCHRIFT

(11) **EP 4 427 213 B1**
(45) Veröffentlichungstag und Bekanntmachung des Hinweises auf die Patenterteilung: **15.04.2026**
(21) Anmeldenummer: 22812652.0
(22) Anmeldetag: 04.11.2022
(51) Int. Cl.: G10H 1/00, G10H 1/40

(54) **VORRICHTUNG UND VERFAHREN ZUR AUSGABE EINES AKUSTISCHEN SIGNALS BASIEREND AUF PHYSIOLOGISCHEN DATEN**
DEVICE AND METHOD FOR OUTPUTTING AN ACOUSTIC SIGNAL ON THE BASIS OF PHYSIOLOGICAL DATA
DISPOSITIF ET PROCÉDÉ D'ÉMISSION D'UN SIGNAL ACOUSTIQUE SUR LA BASE DE DONNÉES PHYSIOLOGIQUES

(30) Priorität: 04.11.2021 AT 508742021
(43) Veröffentlichungstag der Anmeldung: 11.09.2024
(73) Patentinhaber: Graber, Oliver Peter, 2700 Wiener Neustadt (AT)
(72) Erfinder: GRABER, Oliver Peter, 2700 Wiener Neustadt (AT)
(74) Vertreter: Loidl, Manuela Bettina
(86) Internationale Anmeldenummer: PCT/EP2022/080808
(87) Internationale Veröffentlichungsnummer: WO 2023/079073

(56) Entgegenhaltungen:
- JP-A- 2006 251 053
- US-A1- 2004 077 934
- US-A1- 2004 116 784
- US-A1- 2016 098 980

## Beschreibung

### GEBIET DER ERFINDUNG

Die vorliegende Erfindung betrifft ein Verfahren und eine Vorrichtung zur Ausgabe eines akustischen Signals, welches aus akustischen Signalabfolgen aufgebaut ist und wobei der Aufbau des akustischen Signals auf physiologischen Daten beruht.

### HINTERGRUND DER ERFINDUNG

Es ist bekannt, dass Musik in besonderer Weise mit Personen in Resonanz gebracht werden kann, wenn sie mit physiologischen Parametern des Menschen wie beispielsweise der Atem- oder Herzfrequenz (Puls) abgestimmt ist.

Die D1 (US2004077934) beschreibt ein System, das physiologische Parameter - insbesondere die Atmung eines Nutzers - in Klang und Rhythmus umwandelt. Dabei entsteht eine polyphone, mehrschichtige Musik mit mehreren Instrumenten: Eine Grundmelodie wird einer bestimmten Atmungsphase zugeordnet, während zusätzliche Schichten (bis zu vier) dynamisch hinzugefügt werden, um die Musik an die aktuelle Atmungsrate anzupassen. Die Instrumentenwahl richtet sich nach dem Musikstil sowie der Wahrnehmung bei unterschiedlichen Atmungsraten und Inspirations-/ Expirationsverhältnissen. Im Gegensatz zur klassischen Kompositionstheorie ist die erzeugte Musik flexibel in Tempo und Rhythmus, da sie sich direkt an die individuellen Atmungseigenschaften anpasst. EP2113194A1 beschreibt eine Vorrichtung zur akustischen und visuellen Darstellung von physiologischen Daten wie Puls, Hautleitwerte oder Gehirnströme, zur Beeinflussung der Herz- oder Atemfrequenz.

Die Umsetzung physiologischer Parameter von einer elektrischen Schaltung, wodurch ein Ton und ein Rhythmus erzeugt werden, ist in US6662032 beschrieben.

In EP0432152 werden Elektroenzephalogrammdaten in Musik umgesetzt, wobei im EEG-spezifischen Bereich die Gehirnaktivität nur schwer aktiv beeinflusst werden und dieses Biofeedbackverfahren daher sehr ermüdend und langwierig sein kann.

US7177672B2 beschreibt ebenfalls Vorrichtungen zur Messung und Speicherung der Herzschlagintervalle und der Kodierung in Musik, wobei der Rhythmus der Musik mit den gespeicherten Herzschlagintervallen gekoppelt ist.

US2020214615A1 beschreibt ein System, das eine patientenspezifische Audiodatei an einen Patienten zur Therapie oder Rehabilitation überträgt, während der Patient eine Übung durchführt, wobei die Audiodatei mehrere ganze Musiktitel umfasst, gleichzeitig mit einer zweiten Audiodatei mit einer motivierenden Botschaft, und wobei ferner das Tempo des Titels oder die Auswahl der Titel auf der Grundlage von Daten geändert wird, die von Sensoren empfangen werden.

EP0301790A2 beschreibt ein Verfahren, in dem Signale mittels aufgesetzten, oder implantierten Elektroden erfasst und in digitale Daten umgewandelt werden. Über einen MIDI Code werden dann Musikstücke oder Videos generiert.

EP1128358A1 beschreibt Verfahren zur Generierung von Audioprogrammen basierend auf den Vorlieben des Nutzers.

WO2019231835A1 beschreibt eine Vorrichtung zur Generierung einer Melodie, in der die Emotionen eines Benutzers oder Daten aus deren Umgebung dazu verwendet werden, um eine Melodie aus einer Vielzahl von Melodien auszuwählen, die der jeweiligen Situation der Person entspricht. Die Ermittlung der Emotion als Parameter für die Auswahl der Melodie erfolgt durch die Messung des Gesichtsausdrucks oder Körperhaltung, oder durch Messung von Herzschlag, Puls oder Blutdruck.

US2010192754A beschreibt ein Gerät und ein Verfahren zur Generierung von Musik Files, wobei ein Biosignal des Benutzers erfasst wird und das Biosignal zu einer Musik-Kompositions-Information angepasst wird. Das Biosignal wird ausschließlich mittels Elektrokardiogramm- und Photoplethysmographie Signalen ermittelt, sobald der Befehl zur Generierung eines Musikfiles kommt.

US2016098980A1 beschreibt ein musikerzeugendes System, wobei physiologische und Umgebungsparameter eines Benutzers herangezogen werden.

US2009256801A1 beschreibt ein System, in dem Gesten eines Benutzers erfasst und diese Daten verwendet werden, um Töne eines Instruments zu erzeugen.

US5471009A beschreibt ein Gerät zur Erzeugung von Geräuschen.

JP4496993B2 beschreibt eine Vorrichtung und ein Verfahren, um Klänge zu erzeugen, die den Zustand eines sich bewegenden Körpers widerspiegeln.

US8672852B2 beschreibt eine Vorrichtung und ein Verfahren, um Töne zu erzeugen, die den Ein- und Ausatemvorgang mit jeweils einer zugeordneten Klangfarbe im Sinne eines Biofeedbacks v.a. zur Blutdrucksenkung hörbar machen, ermöglicht dabei jedoch keine kreative Erstellung immer neuer Musikwerke, akustischer Gesamtsequenzen, in Echtzeit, die jeweils nur ein einziges Mal erklingen, darüber hinaus ist das Verfahren nicht für kritisch kranke oder sedierte Personen auf Intensivstationen oder bei maschineller Beatmung einsetzbar.

US2014/0249358A1 beschreibt eine Vorrichtung und ein Verfahren, um Musik auf Basis von HRV (Herzratenvariabilitätsmessungen) an ultra- bzw. circadiane Rhythmen anzupassen bzw. in Abstimmung auf diese auszuwählen, wobei insbesondere Sprache und Wiegenlieder implementiert sind. Atem- oder Bewegungssensoren werden nicht verwendet. US2014/0249358A1 ermöglicht jedoch ebenfalls nicht die kreative Erstellung immer neuer Musikwerke in Echtzeit, die jeweils nur ein einziges Mal erklingen.

Bislang bestehende Verfahren bieten keine Lösung zur sensorgesteuerten algorithmischen Komposition an, die gezielt einen gerichteten musikalischen Formbau und darin einen absichtsvoll gestalteten und klinisch wirksamen musikalischenergetischen Hüllkurvenverlauf realisiert. Die bekannten Verfahren verwenden z.B. Einzeltöne an Stelle von Phrasen, und ordnen dabei bereits bei der Melodiebildung einzelne Tonhöhen beliebigen physiologischen Wertebereichen zu, womit sich eine völlig willkürliche Zuordnung ergibt, wodurch die Ergebnisse akustisch als Sonifikation der Daten, aber nicht als Musik wahrgenommen werden. Insofern ist die Bezeichnung "Musikgenerierung" in vorbestehenden Lösungen oftmals irreführend. Die Verfahren berücksichtigen auch nicht das Einsatzfeld der Intensivmedizin (ICU) bzw. der maschinellen Beatmung inklusive der Entwöhnungsphase von maschineller Beatmung (das sogenannte Weaning), Delir und Neurorehabilitation.

Daher besteht die Aufgabe der vorliegenden Erfindung darin, ein Verfahren und eine Vorrichtung zu Verfügung zu stellen, womit die Resonanzbildung zwischen Personen und Musik optimiert und die Physiologie durch Musik klinisch wirksam moduliert und getriggert werden kann. Die Qualität der ästhetischen Wahrnehmbarkeit des akustischen Endprodukts ist dabei ein wichtiges Merkmal.

### KURZDARSTELLUNG DER ERFINDUNG

Die Erfindung ist in den beigefügten Ansprüchen dargelegt.

### KURZE BESCHREIBUNG DER FIGUREN

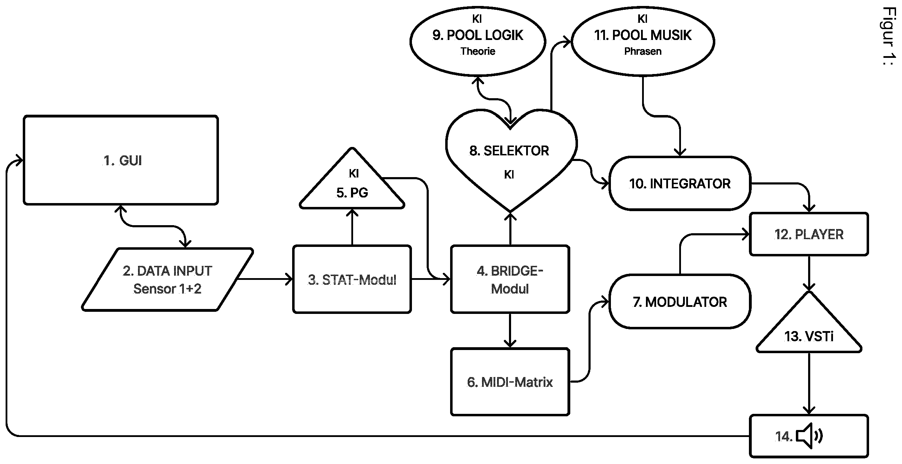

Figur 1: Elemente der erfindungsgemäßen digitalen Systemlösung.

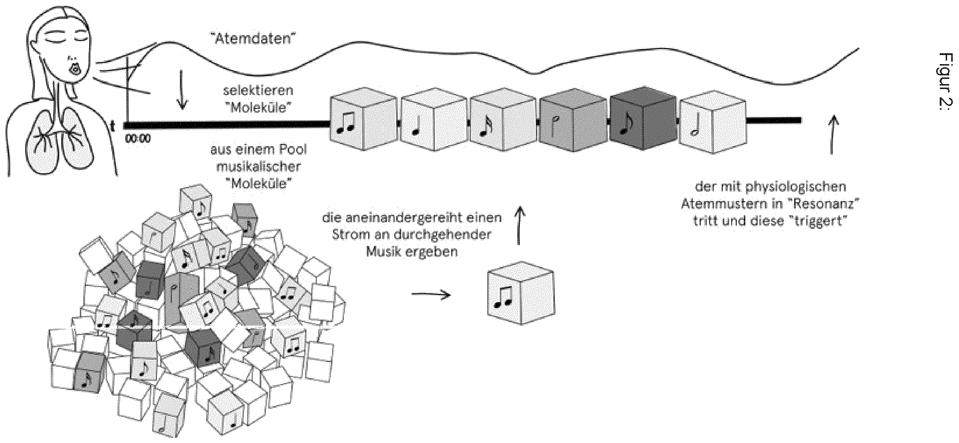

Figur 2: Schematische Darstellung einer Ausführungsform des Verfahrens gemäß der Erfindung. Musikalische "Bausteine" bzw. "Moleküle", (auch "Phrasen" genannt), werden auf Basis physiologischer Daten aus einem Pool solcher "Moleküle" ausgewählt und nach bestimmten musikalischen Regeln adaptiert aneinandergereiht, sodass ein durchgehender musikalischer Fluss entsteht, der resonanzbildende Wirkmuster aufweist.

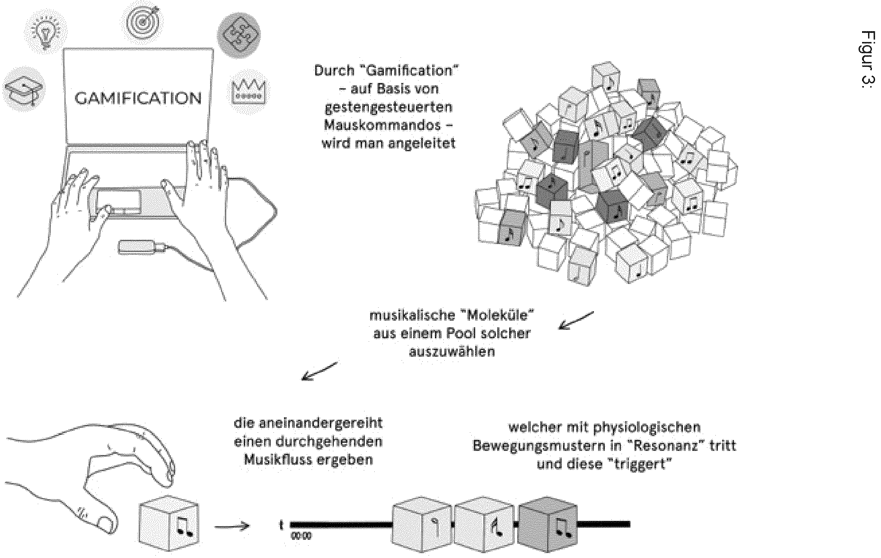

Figur 3: Schematische Darstellung einer Ausführungsform des Verfahrens gemäß der Erfindung. Musikalische "Bausteine" bzw. "Moleküle", auch "Phrasen" genannt, werden auf Basis gestengesteuerter Befehle ausgewählt und nach bestimmten musikalischen Regeln adaptiert aneinandergereiht, sodass ein durchgehender musikalischer Fluss entsteht, der resonanzbildende Wirkmuster aufweist.

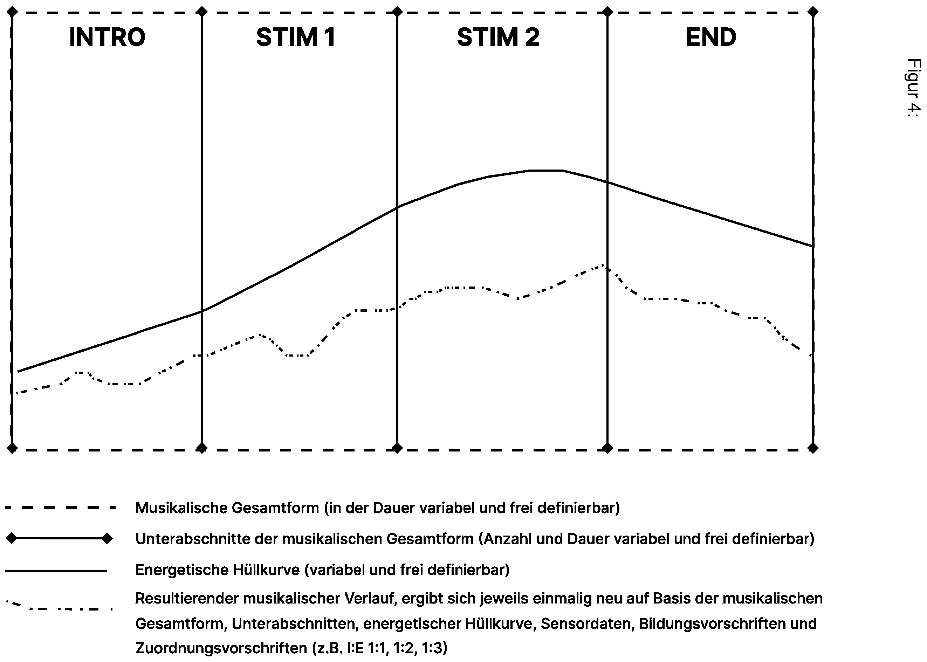

Figur 4: Musikalischer Formverlauf wird festgelegt, indem dessen gesamte Zeitdauer (3 Minuten, 5 Minuten, 10 Minuten, 20 Minuten) und Unterabschnitte (dem klinischen Einsatzfeld entsprechend benannt in INTRO - STIM 1...STIM x - END) bei jeder einzelnen Anwendung aufs Neue definiert werden, wobei für jeden zu bestimmenden Unterabschnitt ein Zeitwert definiert wird, sodass sich die Einzelsummen aller Unterabschnitte zu der vorab festgelegten Gesamtzeit summieren.

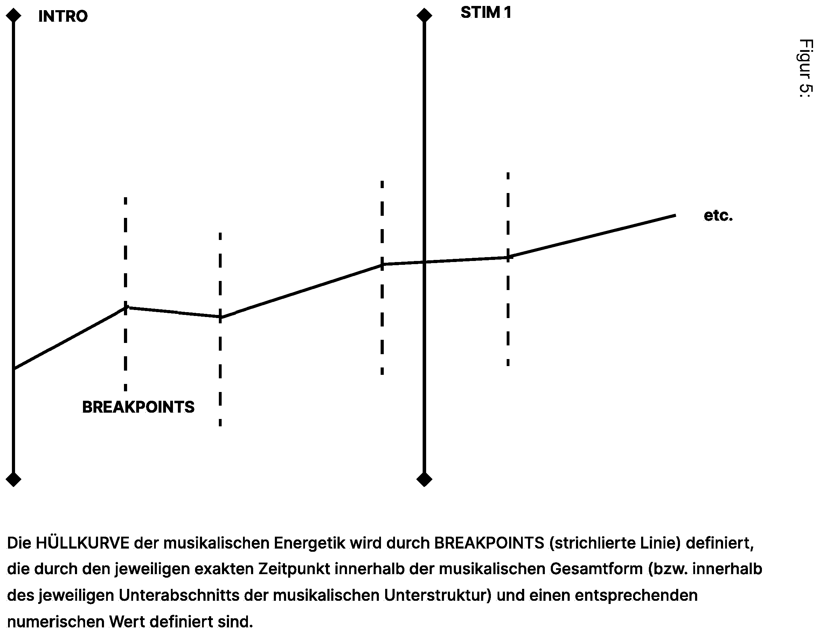

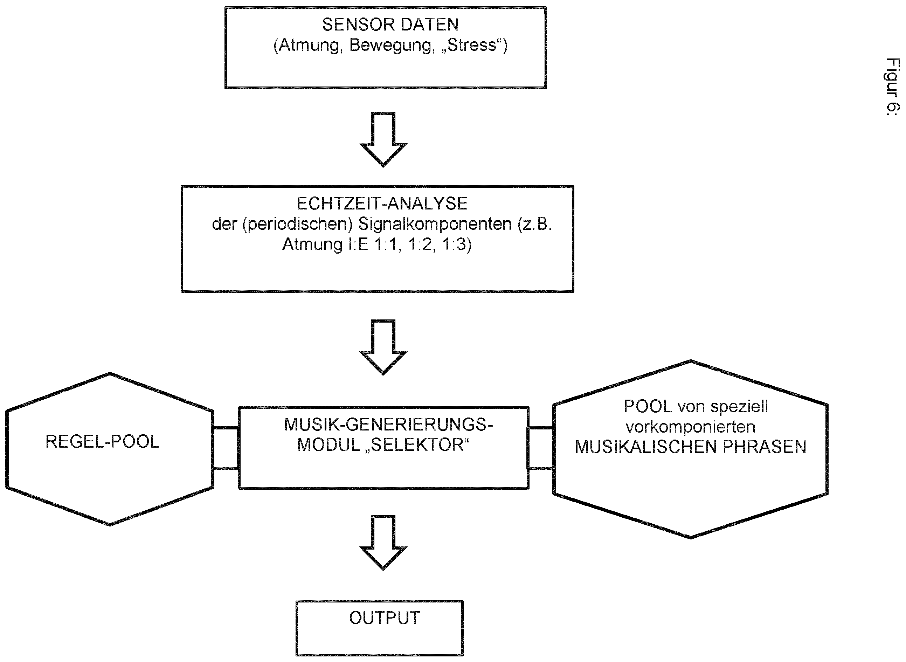

Figur 5: Für die Gesamtheit der musikalischen Form - wie für jeden Unterabschnitt - wird im Zuge dessen zugleich eine energetische Hüllkurve festgelegt. Die dabei zu bestimmenden Werte umfassen zumindest den Zeit- und Amplitudenverlauf der Kurve, indem Breakpoints festgesetzt werden. Der Verlauf der Kurve zwischen den einzelnen Breakpoints ist dabei beliebig formbar. Die Anzahl der Breakpoints ist beliebig, der zeitliche Abstand der Breakpoints ebenso, jedoch ist dieser in Bezug auf den dabei kürzest möglichen Abstand mit der Länge der kürzesten im System vorgehaltenen, in Schritt B (siehe Figur 6 bzw. Beispiel 2) vorproduzierten musikalischen Phrase korreliert.
Figur 6: Flussdiagramm der Schritte A bis E und darin bestehende Abhängigkeiten in Bezug auf den Selektor. Der REGEL-POOL definiert:
   1.) die musikalische Gesamtform
   2.) Zahl und Dauer der Teilabschnitte (musikalische Formteile) der Gesamtform (INTRO, STIM1...STIMx, END)
   3.) den Verlauf der energetischen Hüllkurve
   4.) musikalische Regeln, welche die Kombinierbarkeit der einzelnen Phrasen festlegen.

   Das MUSIK-GENERIERUNGS-MODUL "SELEKTOR" wählt die einzelnen vorkomponierten und gespeicherten Phrasen im Format .mid auf Basis des aktuellen Zeitverlaufs wie ihrer Zuordnung zu musikalischen Formteilen, energetischen Hüllkurvenwerten und physiologischen Signalen (z.B.: I:E 1:1, 1:2, 1:3) aus und fügt diese auf Basis des Pools an musikalischen Regeln zusammen.
   Der POOL von speziell vorkomponierten MUSIKALISCHEN PHRASEN, welche typisch sind für bzw. klassifiziert sind nach
      1.) spezifischen musikalischen Formteilen (INTRO, STIM1...STIMx, END)
      2.) bestimmten Werten der energetischen Hüllkurve
      3.) bestimmten Werten der physiologischen Daten (I:E 1:1, 1:2, 1:3).

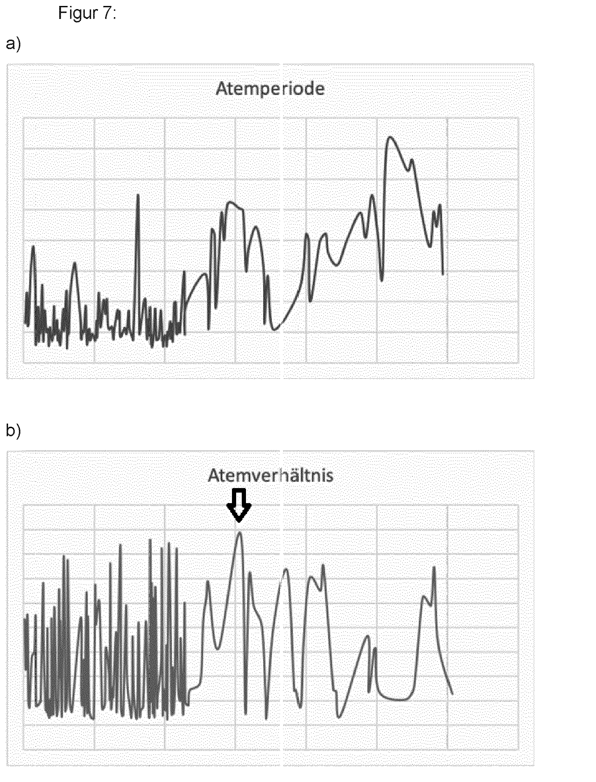

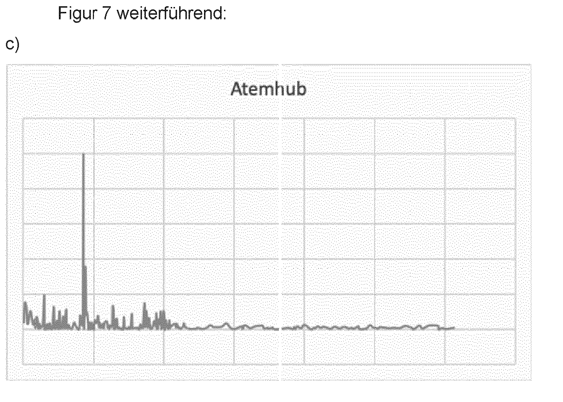

Figur 7: Physiologische Daten eines Patienten erhoben im Rahmen des erfindungsgemäßen Therapieverfahrens auf einer Intensivstation a) Atemperiode, b) Atemverhältnis, c) Atemhub.

### AUSFÜHRLICHE BESCHREIBUNG DER ERFINDUNG

Bei der Erfindung handelt es sich um eine, unter Einsatz von künstlicher Intelligenz (Kl), d.h. in einem selbstlernenden und selbstoptimierenden Computerprogramm realisierte digitale Lösung aus dem Bereich der Computermusik, bei der unter Verwendung von zumindest einem Sensor, wie z.B. von Kamera, Atem- oder Bewegungssensor, speziell komponierte und in ihren Parametern auf den Bestimmungszweck der Resonanzbildung mit physiologischen Daten im Detail abgestimmte funktionelle Musik in ihrem horizontalen wie vertikalen strukturellen Verlauf in Echtzeit generiert und hervorgebracht wird. Dem technischen Stand von MIDI (Musical Instrument Digital Interface) entsprechend, kann diese Musik dabei zudem in Tempo, Lautstärke, Artikulation und Klangfarbe auf Basis der Sensordaten synchron zum physiologischen Geschehen adaptiert werden. Das System kann dabei zum unmittelbaren, aktiven Akt des Musizierens im Zusammenspiel von bis zu zwei Personen verwendet werden.

Die Erfindung stellt insbesondere ein System und eine Methode zur algorithmischen Komposition für den klinischen Einsatz dar und damit ein System und eine Methode zur Erzeugung ästhetisch in hohem Maße ansprechender Musik, die mit dem "physiologischen System" Mensch auf Basis von Resonanzphänomenen koppelbar ist.

Ein klinisch wirksames akustisches Signal, i.e. eine klinisch wirksame Musik, soll auch eine Beschaffenheit aufweisen, welche das Entstehen von Resonanzeffekten, wie z.B. Regulierung der Atmung, Regulierung des Herzschlags, in idealer Weise möglich macht. Dies ist beispielsweise zu erreichen, indem man den energetischen (im Sinn von Ernst Kurth) Grad und Gehalt von Musik auf die Aufnahmefähigkeit von Personen (sedierte Personen nehmen anders auf und wahr als wache) bzw. auf spezifische Aspekte deren jeweiligen physiologischen Situationen, die sich im klinischen Umfeld, v.a. auf Intensivstationen zudem pathologisch beeinflusst darstellen, abstimmt, indem man diese z.B. mit atmungs- und bewegungsspezifischen Parametern möglichst in Übereinstimmung bringt. Ein Beispiel ist, wenn die Herzrate bei 72 beats per minute liegt, ein Musiktempo von 72 bpm zu wählen.

Bei mechanisch beatmeten Personen ist die Musik auch auf die Funktionsweise der Beatmungsmaschine und das Stadium der Entwöhnung von der maschinellen Beatmung (dieser Prozess wird Weaning genannt) abzustimmen.

Insbesondere erlaubt die vorliegende Erfindung die Erzielung von
a. einer spezifischen musikalischen Form
b. einem dafür erforderlichen, energetisch-musikalisch gestalteten Verlauf der musikalischen Gesamtheit und damit einer energetischen Hüllkurve musikalischen Zeitverlaufs wie aller Unterabschnitte und sämtlicher musikalischen Parameter
c. Melodik und Phrasenbildung der resultierenden Musik, die in Übereinstimmung mit physiologischen Parametern des Atmens, bzw. der Bewegung steht und diese akustisch repräsentiert
d. dem Einsatz und der Kombination von musikalischen Teilphrasen statt Einzeltönen und
e. der Einbeziehung von Aspekten der maschinellen Beatmung.

Ein besonderes Merkmal der Erfindung ist der ästhetische, funktionell-kompositorische Aspekt und damit die sensorgesteuerte Generierung von "horizontalem" musikalischem Verlauf (d.h. in der Zeitachse wie daraus resultierend musikalischer Form) in Echtzeit. Zu diesem Zweck hält das System keine kompletten Musikstücke im Format .mid (MIDI) vor, sondern musikalische Teilstücke, auch Signalabfolgen oder Phrasen genannt, die auf Basis der Sensordaten zu einem Gesamtwerk, i.e. einer Gesamtsequenz, dem akustischen Signal, zusammengefügt werden. Des Weiteren übernimmt die KI selbstlernend direkt die Erzeugung der Musik, d.h. die Erzeugung eines finalen akustischen Signals, im Gesamten auch "Adaptomorphe Musik" genannt.

Daneben können in der Kombination von Atem- und Bewegungssensor besonders effektive Einsatzmöglichkeiten entstehen, die auch das Musizieren zu zweit einschließen. Somit kann bei jeder Sitzung ein neuer, ästhetisch anspruchsvoller, musikalischer (Gesamt-)Verlauf, i.e.das Klangbild, das akustische Signal, die akustische Gesamtsequenz, generiert werden, der nur ein einziges Mal erklingt. Damit ist die erfindungsgemäße Vorrichtung ein digitales System zur funktionellen Komposition und zugleich ein digitales Musikinstrument.

Der Begriff "akustisches Signal" oder "akustische Gesamtsequenz" oder "akustisches Gesamtsignal" bezieht sich gemäß der Erfindung auf eine akustische Entität (Musikstück), das aus mehreren akustischen Signalabfolgen zusammengesetzt ist. Ein akustisches Signal wird bestimmt durch das Ergebnis der Kategorisierung der Parameter physiologischer Daten wie das Verhältnis von Ein- und Ausatmung, Atemfrequenz, Atemperiode, Atemhub oder spezifische Bewegungen der Finger, Hände und Arme und deren zeitlichen Trends, wobei die einzelnen Kategorien der Parameter ausgewählten akustischen Signalabfolgen zugeordnet werden.

Der Begriff "akustische Signalabfolge" bezieht sich auf kurze, aneinandergereihte akustische Sequenzen von etwa 0,5 bis 70 Sekunden.

Der Begriff "Teilsignal" bezieht sich auf musikalische Bausteine, auch "musikalische Moleküle" oder "Phrasen" genannt, die aus zumindest, jedoch nicht ausschließlich, zwei Tönen bestehen.

Unter "Kategorisieren" der Parameter physiologischer Daten im Zusammenhang mit der Erfindung wird verstanden, die Teilsignale und/oder die Signalabfolgen einem Verhältnis der physiologischen Parameter, beispielsweise I:E im Verhältnis 1:1, 1:2, 1:3, etc., der energetischen Hüllkurve und musikalischen Teilabschnitten (INTRO, STIM1... STIMx, END) zuzuordnen. Jeder dieser Teilabschnitte kann beispielsweise eine Dauer von etwa 20 Sekunden bis 9 Minuten haben. Im Speziellen kann die Dauer 20, 30, 40, 50, oder 60 Sekunden, oder 1, 2, 3, 4, 5, 6, 7, 8, oder 9 Minuten betragen.

Unter "Diskretisierung" und "Quantisierung" im Zusammenhang mit der Erfindung wird die Umwandlung eines Analogsignals in ein Digitalsignal verstanden, wobei zu definierten Zeitpunkten eine Abtastung erfolgt und die Abtastwerte als Zahlenwerte dargestellt werden. Digitale Werte sind üblicherweise als Binärzahlen kodiert. Ihre Quantisierung wird somit in Bits angegeben. Die von den Sensoren gelieferten Daten werden numerisch erfasst und statistisch insbesondere hinsichtlich ihrer Trends über bestimmte Zeiträume ausgewertet. Zudem erfolgt eine Skalierung auf den im MIDI-Protokoll verankerten numerischen Wertebereich (0-127).

Unter dem Begriff MIDI ist eine digitale Schnittstelle für Musikinstrumente ("Musical Instrument Digital Interface") zu verstehen. Es ist ein Datenübertragungsprotokoll, das u.a. die Kommunikation zwischen einem (Personal-) Computer und verschiedenen Instrumenten ermöglicht. MIDI ist der Industriestandard für den Austausch von musikalischen Steuerinformationen und Daten zwischen elektronischen Musikinstrumenten.

Das MIDI-Protokoll wird derzeit von vielen Soundkarten in Personal-Computern unterstützt. Somit stellt das MIDI-Protokoll keine Audiosignale bereit, sondern besteht aus Befehlen zur Ansteuerung von Instrumenten oder der Soundkarte. Der entsprechende Befehl, der übermittelt wird, kann beispielsweise derart ausgestaltet sein, dass dieser etwa "Note-on" (schalte Note an), "Velocity" (gib die Anschlagstärke an) und "Note-off' (schalte Note aus) heißt. Diese Steuerbefehle werden an einen Klangerzeuger, beispielsweise eine Soundkarte, einen Synthesizer, Sampler, oder eine entsprechend aufbereitete Klangbibliothek, die aus unterschiedlichen Instrumentenklängen besteht, gesendet.

Der Begriff "Adaptive Musik" bezeichnet Musik für ein und in einem Computerspiel (Game-Music), die in jeder Hinsicht flexibel sein muss, da nicht im Vorhinein bekannt ist, wie lange sich eine Person in einer bestimmten Spielsituation aufhält. Dies ist von dem hier verwendeten Begriff "adaptomorph" zu unterscheiden, der die Gesamtformgebung mit der Silbe "morph" unterstreicht.

Funktionelle Musik bezeichnet Musik, die durch außermusikalische Einflüsse bestimmt und solchermaßen für spezielle Einsatzzwecke gedacht ist oder dafür erstellt wird. Der Vorgang der Schöpfung solcher Musik wird als funktionelle Komposition, als funktionelles Komponieren bezeichnet.

Der Begriff "musikalischer Formteil" oder "musikalischer Teilabschnitt" beschreibt strukturbildende Unterabschnitte eines Musikwerkes. Musikalische Form wird auch als "musikalische Architektur" bezeichnet.

Der Begriff "Energetik" (auch musikalische Energetik genannt) bezieht sich auf die Forschungsergebnisse des Musiktheoretikers und Musikpsychologen Ernst Kurth und ist u.a. ein Maß dafür, wie ereignisreich sich Musik gestaltet. Entscheidend für die Erfindung ist es dabei, bei größtmöglicher Übereinstimmung mit dem sensorerfassten physiologischen Geschehen einen jeweils frei definierbaren, klinisch wirksamen energetischen Verlauf innerhalb der Gesamtform wie in deren einzelnen musikalischen Formteilen, auch Teilabschnitte genannt, zu gewährleisten.

Der Begriff "Klangbild" beschreibt die Gesamtheit des jeweils Erklingenden, einschließlich aller dabei imponierenden Sinnes-Eindrücke wie Tonfolgedichte, Ambitus, Musikstil oder Klangfarbe, etc.

Der Begriff "Improvisation" (musikalische Improvisation) beschreibt einen spontanen kreativen Akt der Hervorbringung eines neuen musikalischen Werkes, eines neuen akustischen Signals. Improvisationen am Krankenbett mit oder ohne Patientenbeteiligung zählen zu den wichtigsten Arbeitsmethoden im Rahmen der Musiktherapie. Improvisationen entstehen aus dem Moment heraus und erklingen nur ein einziges Mal. Die gegenständliche Erfindung simuliert diese Herangehensweise der Musiktherapie und stellt somit einen digitalen Musiktherapie-Assistenten dar.

Der Begriff "harmonische Fortschreitung" umfasst Regeln, wie von einem zum nächsten Akkord fortzuschreiten ist. Nach William E. Caplin sind dabei die prolongierende Fortschreitung, bei der eine harmonische Funktion über die Interpolation untergeordneter Nebenharmonien hinweg aufrechterhalten bleibt, die kadenzielle Fortschreitung aus Anfangstonika, "Prädominante" (etwa ein Akkord der Subdominante = IV. Stufe, Subdominantparallele = II. Stufe oder Tonikaparallele = VI. Stufe), Dominante und Schlusstonika als Bestätigung einer Tonalität und die sequenzielle Fortschreitung, bei der die Harmonien nach einem regelmäßigen Schema von Grundton-Bewegung und kontrapunktischer Stimmführung organisiert sind, zu unterscheiden. Die Begriffe Tonika, Subdominante und Dominante etc. bezeichnen bestimmte Tonstufen im Rahmen der Dur-Moll-tonalen Musik, die kompositionstechnisch einer bestimmten Abfolge bzw. Behandlung bedürfen. Diese der Dur-Moll-tonalen Musik entnommenen Beispiele dienen an dieser Stelle nur der Verdeutlichung des Begriffes und schränken die Erfindung, die unter anderem auch modale, chromatische, außereuropäische Tonsysteme umfasst, in keinster Weise ein.

Der Begriff "Ambitus" beschreibt den Tonumfang etwa einer Melodie, gemessen vom tiefsten bis zum höchsten darin auftretenden Ton.

Der Begriff "Modulation" beschreibt den absichtsvoll und mit gezielten Mitteln vollzogenen, eineindeutigen Wechsel von einer Tonart in eine andere.

Der Begriff kontrapunktische Stimmführung (Kontrapunkt) beschreibt das Führen musikalischer Linien (Melodien), Motive oder Themen im regelgerechten Verhältnis zueinander.

Klänge bzw. Klangfarbe meint im Zusammenhang mit der Erfindung die Zuordnung einzelner MIDI-Signale zu bestimmten Instrumentenklängen (digitale Instrumentation bzw. Orchestration) z.B. in einem VSTi oder die Wiedergabe von Samples in beliebigen Audio-Formaten vermittels eines VSTi.

Der Begriff "Geste(n)" oder "Bewegungen" meint im Zusammenhang mit der Erfindung Bewegungen der Hände, die durch Therapieabläufe im Bereich der Neurorehabilitation [Flexion und Extension (Ellbogengelenk), Pronation und Supination (Unterarm), (großer) Faustschluss (Flexion Fingergelenke bzw. Opposition Daumen), "Klopfbewegungen" (Palmarflexion und Dorsalextension im Handgelenk), Zueinanderführen der jeweiligen Fingerkuppen (Fingerschluss) beider Hände in den Kombinationen Daumen-Zeigefinger | Daumen-Mittelfinger | Daumen-Ringfinger | Daumen-Kleiner Finger] oder künstlerische Bewegungsabläufe (z.B. Dirigierbewegungen) hinsichtlich ihrer Durchführung eindeutig definiert und international standardisiert sind.

Der Begriff "physiologische Daten" bezieht sich auf das Ergebnis von physiologischen Messungen wie Dauer der Ein- und Ausatmung, Atemfrequenz, Atemperiode, Atemhub oder spezifische Bewegungen der Finger, Hände und Arme, die durch entsprechende Sensoren ermittelt werden können.

Im hier beschriebenen Verfahren kann bei physiologischen Daten mit periodischem Verlauf die Periodendauer als Parameter herangezogen werden. Beispielsweise kann eine Periodendauer 1, 2, 3, ... x Atemzüge umfassen oder x-Mal 60 Sekunden/Atemfrequenz pro Minute lang sein.

Ein Verlauf der kategorisierten Parameter kann beispielsweise festgelegt werden, indem eine Tendenz festgestellt wird, ob die physiologischen Parameter gegenüber dem Startpunkt der Erfassung unverändert bleiben, sich verbessern oder verschlechtern. Dazu wird mit aus der Fachliteratur bekannten physiologischen Normwerten abgeglichen.

Sensoren sind Instrumente, die physiologische Parameter (Signale) erfassen und sie in elektrische bzw. digitale Signale umwandeln. Nicht limitierende Beispiele für Atemsensoren sind aus dem Stand der Technik bekannt, wie etwa Atemfühler, Thermal Atemsensoren, Atemsensoren mit Druck-Transducer zur Erzeugung einer Ausgangsstromspannung, die in Relation zum Luftdruck steht, Atemsensoren bei denen der Atemfluss durch einen Piezosensor aufgenommen wird, Atemsensoren bei denen das Signal durch Radar erfasst wird, und Atemsensoren, bei denen die Atemaktivität durch Zugkräfte auf einen Fühler (Brustgurt) bestimmt wird.

Relevant für die vorliegende Erfindung ist, dass kein Biofeedback-System realisiert wird, d.h. das finale akustische Signal ist nicht durchgehend 1:1 zur Atmung synchronisiert.

Nicht einschränkende Beispiele für Bewegungssensoren sind Leap Motion Bewegungssensoren, 3D Gestencontroller, beispielsweise basierend auf GestlC-Technologie, 3D Infrarot Gestencontroller oder Bewegungserkennung via Kamera.

Die Herzfrequenz ist die Anzahl der Herzschläge pro Minute. Die Herzschlagfrequenz oder Herzfrequenz wird häufig mit dem Puls gleichgesetzt. Eine Messung kann beispielsweise durch ein Elektrokardiogramm (EKG) erfolgen.

Der Begriff "Parameter" physiologischer Daten umfasst messbare körpereigene Signale, die mit psychischen, mentalen oder Organfunktionen zusammenhängen. Diese Signale werden durch Sensoren erfasst und numerisch und methodisch analysiert, diskretisiert, parametrisiert und kategorisiert.

Der Begriff "Atemperiode" bezieht sich auf den Zyklus bestehend aus einer Ein- und einer Ausatmung, die Länge der Atemperiode ergibt sich mathematisch aus folgender Beziehung: 60 Sekunden/Atemfrequenz pro Minute.

Der Begriff "Atemhub" bezieht sich auf das Volumen der pro Zyklus/Atemzug eingeatmeten Luft.

Die Atemfrequenz beschreibt die Anzahl der Zyklen/Atemzüge pro Minute.

"Echtzeit" gemäß der vorliegenden Erfindung bezieht sich auf die Generierung des akustischen Signals innerhalb weniger Sekunden.

Der Begriff "Bildungsvorschrift" bezieht sich auf die spezifische Struktur der für die Datenbank speziell komponierten musikalischen Teilstücke, sodann auf Regeln, welche Zusammenhänge zwischen den physiologischen Daten und der Auswahl und Beschaffenheit der musikalischen Teilstücke herstellen und andererseits auf musiktheoretische Regeln, welche die Abfolge der Teilstücke auf rein musikalischem Sektor bestimmen.

Die Regeln welche sich aus den Gesetzmäßigkeiten der europäischen und/oder außereuropäischen Kunstmusik ableiten sind beispielsweise die Abfolge bestimmter Tonstufen oder Intervalle, wie etwa die Abfolge von Tonika, Subdominante und Dominante. Besonders geeignet sind dabei beispielsweise musikalische Strategien, die auf musikalischen Traditionen der griechischen und persischen Antike, dem Gregorianischen Gesang und der Kompositionstechnik von A. Vivaldi, J.-S. Bach, G.-F. Händel, J. Haydn, W.A. Mozart, L.v. Beethoven, A. Reicha, F. Liszt und C. Debussy basieren.

Die hier beschriebene "Datenbank" umfasst eine Vielzahl von speziell gefertigten musikalischen Teilstücken, i.e. akustische Teilsequenzen, die sich stilistisch an europäischer und außereuropäischer Kunstmusik orientieren, aus denen das akustische Signal gebildet werden kann. Diese Teilstücke können im Format .mid (MIDI) vorrätig gehalten sein (POOL MUSIK, (11)). Die Länge dieser Teilstücke kann 0,5 bis 70 Sekunden betragen. Zudem existiert eine weitere "Datenbank" mit einer Vielzahl an musikalischen Regeln zur Verknüpfung der Teilstücke (POOL LOGIK, (9)).

Die Erfindung umfasst auch eine Vorrichtung zur Durchführung der erfindungsgemäßen Verfahren enthaltend zumindest einen Sensor wie etwa einen Atemsensor, einen Gestencontroller oder eine Kamera zur kontinuierlichen Erfassung ausgewählter physiologischer Daten, wie etwa das Verhältnis von Ein- und Ausatmungsdauer oder Bewegungen der Hände, Mittel zur Diskretisierung, Quantisierung und Kategorisierung des Verlaufs vorgegebener Parameter der physiologischen Daten (STAT-Modul, (3)), Mittel zur Zuordnung akustischer Signalabfolgen zu den Kategorien (SELEKTOR, (8)) und Mittel zur Kombination der akustischen Signalabfolgen entsprechend dem Verlauf der kategorisierten Parameter der physiologischen Daten zu einem akustischen Signal und zur Ausgabe des Signals, welches zu einem finalen Klangbild geformt wird (INTEGRATOR, (10)).

Die Vorrichtung für die Durchführung des erfindungsgemäßen Verfahrens kann beispielsweise folgende Elemente umfassen:
Einen Atem- oder Bewegungssensor
Einen handelsüblichen Computer mit folgenden Programmelementen (1) bis (13)
GUI (1): Graphisches Interface, mit Eingabefeld, zur Kommunikation mit dem Anwender
Sensor (DATA INPUT) (2): als Schnittstelle zum Atemsensor, Bewegungssensor, etc.
STAT MODUL (3): zur Übernahme der Sensordaten und deren statistischen Auswertung
BRIDGE MODUL (4): zur Umwandlung der statistischen Daten in MIDI Befehle
POOL GESTEN (PG, (5)): als Ergänzung zum Bridge Modul, Identifizierung der spezifischen Geste
MIDI MATRIX (6): zur Zuordnungseinstellung in Bezug auf die MIDI Befehle
MODULATOR (7): zur Adaption der MIDI Befehle
SELEKTOR (8): auf Basis der Sensordaten bzw. deren statistischer Auswertung trifft der SELEKTOR die Entscheidung, welches Musikteilstück ausgewählt und abgespielt wird. Die Regeln für die Auswahl der Teilstücke sowie für deren Kombinierbarkeit werden im Zuge des vorausgehenden kompositorischen Prozesses bei der Herstellung der speziellen musikalischen Teilstücke für das System definiert. Der SELEKTOR greift im Sinne eines Expertensystems auf diese Regeln zu, das im POOL LOGIK (9) realisiert ist.
POOL LOGIK (9): enthält die musikalischen Regeln, auf die der SELEKTOR (8) zugreift, inklusive aller Details zu den zumindest vier musikalischen Formteilen und der energetischen Hüllkurve.
INTEGRATOR (10): zur Sicherstellung des entsprechenden Gesamtablaufes auch hinsichtlich des Tempos und weiterer musikalischer Parameter
POOL MUSIK (11): Datenbank enthaltend speziell gefertigte musikalische Teilstücke
PLAYER (12): zur Erzeugung eines MIDI-Datenstroms
VSTi (13): Virtual Studio Technology Instrument. VST ist eine Programmierschnittstelle und darin Industriestandard für Audio PlugIns, etabliert 1996 von der Firma Steinberg Media Technologies. VSTis sind virtuelle Instrumente im Rahmen dieser Schnittstelle. Beispielsweise kann als VSTi der Industriestandard NI Kontakt 7 sowie die Engine (2.7) von Best Service verwendet werden, im Prinzip kann aber jedes VSTi, das windowslauffähig ist, Verwendung finden. Die Klänge, die im Rahmen der Erfindung zum Einsatz kommen, werden speziell für das jeweilige VSTi erstellt/programmiert ("Sounds"/"Presets"). Die Klangprogrammierung ist integraler Teil der erfindungsspezifischen Komposition.

Einen Lautsprecher (14), beispielsweise ein handelsüblicher Lautsprecher oder Kopfhörer/Stöpselhörer/Noise-Cancelling Kopfhörer.

Anwendung findet die Erfindung auch im Bereich der Computermusik.

Eine Hüllkurve (envelope) ist im Rahmen der elektronischen Klangerzeugung ein Mittel zur Klangformung. Mittels Hüllkurven wird etwa bestimmt, wie schnell und stark ein Klang in puncto Lautstärke, Filterfrequenz etc. an- und abschwellen soll. Der Konvention entsprechend wird bei der graphischen Darstellung einer Hüllkurve die Zeit auf die x-Achse gesetzt. Auf der y-Achse wird der Wert eines Parameters von seinem Minimum bis zum Maximum dargestellt. Vielfach werden dabei vier "Breakpoints" definiert und beschrieben: A = Attack (Anstieg), D = Decay (Abfall), S = Sustain (Halten) und R = Release (Freigeben). Der Breakpoint Attack bestimmt, wie lange es dauern soll, bis der steigende Wert eines Parameters ein bestimmtes Niveau erreicht. Decay bestimmt, wie lange es dauern soll, bis der fallende Wert eines Parameters das Niveau des Sustains erreicht. Sustain bestimmt das Niveau eines Parameterwerts, das so lange konstant bleibt, bis das entsprechende Steuersignal endet. Release bestimmt, wie lange es dauern soll, bis der fallende Wert eines Parameters ein bestimmtes Minimum erreicht. In aktuellen Softwareinstrumenten stehen darüber hinaus Hüllkurven mit mehr als diesen vier ADSR-Breakpoints zu Verfügung.

Der Begriff der energetischen Hüllkurve nimmt diesen Begriff und Ansatz auf, wendet ihn jedoch nicht wie bislang üblich auf die Klangformung im Rahmen elektronischer Instrumente an, sondern neu auf die kompositorische Struktur und algorithmische Komposition von Musik an sich. Die einzelnen Breakpoints auf der energetischen Hüllkurve im Sinne der Erfindung (Fig. 5) bestimmen dabei Zeitpunkt und Intensität des musikalischen Ereignisreichtums (der "Energiedichte") der Musik. Dieser ist dabei vor allem, jedoch nicht ausschließlich, durch die Tonfolgedichte und den Ambitus der jeweils zu diesem Zeitpunkt generierten Musik wie darin auftretende Intervalle, Akkorde, Modulationen (Wechsel der Tonarten) und Rhythmen etc. definiert. Durch die energetische Hüllkurve im Sinne der gegenständlichen Erfindung wird also die zu generierende Musik als solche in ihrer Struktur bzw. Machart und nicht ein elektronischer Klang in seinem Verlauf bestimmt. Breakpoint 1 der energetischen Hüllkurve bestimmt demgemäß, welche Ereignisdichte an diesem Zeitpunkt in der Musik herrschen soll und so fort. Die Anzahl der setzbaren Breakpoints ist dabei beliebig, korreliert jedoch im zeitlichen Abstand mit dem kürzesten im System vorgehaltenen akustischen Teilsignal (Phrase), dessen Zeitdauer die unterste Grenze für den Abstand bildet, in dem Breakpoints auf der x-Achse gesetzt werden können.

Dabei dient die Erfindung der atemgestützten Steuerung von MIDI-fähigen Geräten oder der musikalischen Komposition, Improvisation und Interpretation.

Beispiel: Ein digitales Musikinstrument soll über die Atmung in seinem Klangverhalten beeinflusst werden, wobei sowohl die Ein- wie auch die Ausatmung sowie deren Verhältnis und Periodendauer als Steuersignale verwendet werden sollen.

Die Erfindung kann auch der Unterhaltung und Entspannung dienen. Beispiel: Eine Person möchte abends zur Entspannung vor dem Einschlafen ruhige Musik genießen und sucht bei jedem Hörerlebnis größtmögliche musikalische Vielfalt bei identer musikalischer Charakteristik.

Die Erfindung dient auch der bewegungsgestützten Steuerung von MIDI-fähigen Geräten oder der musikalischen Komposition, Improvisation bzw. Interpretation.

Beispiel: Während einer Performance soll das Bühnenlicht durch bestimmte Gesten gesteuert werden.

Die Erfindung kann auch der Unterhaltung und Entspannung mehrerer Personen dienen. Beispiel: Ältere Personen musizieren mit Kindern spielerisch im Duo. Wer sich die Bewegungsfolgen, die zur richtigen Musikwiedergabe führen, rascher merkt, hat gewonnen (Music-Movement-Memory).

Die erfindungsgemäße Vorrichtung dient dem Einsatz in der Musiktherapie in all ihren Erscheinungsformen und ermöglicht durch den kombinierten Einsatz von atem- und bewegungsrelevanten Komponenten durch Entrainment einen sehr hohen Wirkungsgrad.

Beispiel: Studierende der Musiktherapie bereiten sich durch Einsatz des Systems auf ihre berufliche Laufbahn im Bereich Intensivmedizin und Neurorehabilitation vor.

Die Erfindung dient dem Einsatz im Bereich der musikalischen Komposition, Improvisation und Interpretation und ermöglicht durch den kombinierten Einsatz von atem- und bewegungsrelevanten Komponenten einen sehr natürlichen künstlerischen Prozess.

Beispiel: Bei einer Bühnenperformance werden Licht- und Toneffekte durch bestimmte Gesten der Hand ausgelöst, die in ihrer Intensität durch die bewusst gelenkte Atmung der steuernden Person moduliert werden.

### Pädagogik

Die Erfindung dient der Ausbildung von MusikerInnen und TherapeutInnen und ermöglicht durch den kombinierten Einsatz von atem- und bewegungsrelevanten Komponenten eine abwechslungsreiche, mehrschichtige Aufgabenstellung.

Beispiel: Studierende im Fach Dirigieren haben Probleme mit der richtigen Atmung beim Auftakt. Dazu wird mit dem erfindungsgemäßen Verfahren geübt - die dirigentischen Gesten der Hand werden jedoch nur dann vom System als Trigger zugelassen, wenn zuvor richtig eingeatmet wurde.

Das erfindungsgemäße System kann auch der Unterhaltung und Entspannung dienen und durch den kombinierten Einsatz atem- und bewegungsrelevanter Parameter einen entsprechenden Abwechslungsreichtum bieten.

Beispiel: Zwecks Zeitvertreibs kreiert eine Person spielerisch Musik mit dem System durch Gesten, dazu parallel wird die Raumbeleuchtung durch die Atemaktivität moduliert. Das Ziel ist es, den Fluss der Musik und den Wechsel des Farblichtes in ein bestimmtes Verhältnis zu setzen.

### BEISPIELE

Die hier beschriebenen Beispiele dienen der Veranschaulichung der vorliegenden Erfindung und sind nicht als Einschränkungen derselben zu verstehen. Es werden verschiedene Ausführungsformen der vorliegenden Erfindung beschrieben. Viele Änderungen und Variationen können an den hierin beschriebenen und abgebildeten Techniken vorgenommen werden, ohne vom Umfang der Erfindung abzuweichen.

### Beispiel 1

Die erfindungsgemäße Vorrichtung kann für Einsatzbereiche, in denen Drahtlosverbindungen aus Sicherheitsgründen nicht möglich sind, beispielsweise auf Intensivstationen, auf mobilen Endgeräten wie beispielsweise Windows-Tablets ausgeführt werden. Des Weiteren ist eine serverbasierte Version möglich, sodass beliebige Endgeräte benützt werden können.

Beispielhafte Hardwarekomponenten können sein:
1 Atemsensor (Bluetooth) | 1-2 Bewegungssensor(en) (leap motion oder Webcam) | 1 USB-Bluetooth Audiosplitter (alternativ y-Kabel) | 2 Bluetooth Kopfhörer (alternativ kabelgebundene Verbindung zu den Kopfhörern oder ein Lautsprecher-System)

In Figur 1 werden die Elemente der erfindungsgemäßen digitalen Systemlösung dargestellt.

Die Daten der Sensoren werden über DATA INPUT (2) in die Software übernommen, verschiedentlich statistisch ausgewertet unter Zuhilfenahme des STAT-Moduls (3) bzw. als spezifische Geste identifiziert, PG = POOL GESTEN (5) und in MIDI-Befehle gewandelt (BRIDGE Modul, (4)). Die gesamte Kommunikation mit den AnwenderInnen erfolgt über ein grafisches Interface, GUI (1).

Die MIDI MATRIX (6) ermöglicht Zuordnungseinstellungen in Bezug auf die ausgegebenen MIDI-Befehle (insbesondere MIDI CC-Daten), die vom MODULATOR (7) musikalisch sinnstiftend weiter adaptiert werden können, ehe sie zusammen mit den zentralen Sequenzer-Daten (Note on/off Befehle etc.) vom PLAYER (12) über virtuelle MIDI-Kabel an das VSTi (13)-Softwareinstrument weitergeleitet werden, welches schließlich den Audiodatenstrom erzeugt, der von der Abhöre, dem LAUTSPRECHER (14) wiedergegeben wird.

Als wesentliches Element der Erfindung wird die Musik speziell für die Anwendung komponiert, die Kompositionen, d.h. die finalen akustischen Signale, unterliegen dem Urheberrecht. Realisiert werden jedoch nicht komplette ("fertige") Musikstücke, sondern musikalische Teilstücke, die sogenannten akustischen (Teil-)Signale / akustischen Signalabfolgen / Phrasen, die miteinander nach bestimmten Regeln, die sich sowohl aus physiologischen, d.h. Sensordaten wie musikalischen Gesetzmäßigkeiten ergeben, kombiniert bzw. aneinandergereiht werden können, um die fertige Komposition, das finale akustische Signal, zu formen. Dabei kann auch Musik unterschiedlicher Kulturkreise berücksichtigt werden. Für eine besondere Ausführungsform der Erfindung wird die Musik, d.h. die musikalischen Teilstücke zudem so aufbereitet, dass sowohl einhändige, zweihändige und sequentielle Folgen von einer und zwei Personen möglich sind. Die resultierenden musikalischen Teilstücke, die Signalabfolgen, werden im POOL MUSIK (11) im Format .mid (MIDI) vorrätig gehalten. Ein wesentliches Element ist der SELEKTOR (8), welcher auf Basis der Sensordaten bzw. deren statistischer Auswertung die Entscheidung trifft, welches Musikteilstück, d.h. welche Signalabfolge ausgewählt und abgespielt wird. Der SELEKTOR (8) ist integraler Teil des kompositorischen Gesamtkonzeptes der Erfindung. Die Regeln für die Auswahl der Teilstücke, der Signalabfolgen, wie für deren Kombinierbarkeit werden im Zuge des kompositorischen Prozesses definiert. Der SELEKTOR (8) greift im Sinne eines Expertensystems auf diese Regeln zu, das im POOL LOGIK (9) realisiert ist. Dort werden dem Selektor auch alle Informationen zu der musikalischen Gesamtform, wie ihren Unterabschnitten und der energetischen Hüllkurve pro Sitzung definiert, bereitgestellt. Der INTEGRATOR (10) stellt schließlich den ästhetisch ansprechenden Gesamtablauf auch hinsichtlich des Tempos und weiterer musikalischer Parameter sicher. Das eigentliche Abspielen/Generieren der MIDI-Daten erfolgt wie bereits im vorherigen Absatz beschrieben durch den MIDI-Sequenzer PLAYER (12). Die Klangfarben/Klänge ("Sounds"/"Presets"), die das VST-Instrument für die Wiedergabe verwendet, werden im Zuge des Kompositionsprozesses speziell erstellt/programmiert und sind integraler Bestandteil der Komposition. Diese werden direkt im VST-Instrument als "Presets" im Rahmen einer Klangbibliothek vorrätig gehalten.

Das Programmelement der künstlichen Intelligenz ist in die Bereiche SELEKTOR (8), [POOL LOGIK (9), POOL MUSIK (11) und POOL GESTEN (5) integriert und erfüllt folgende Aufgaben:
1.) Überwachung und Optimierung der Anwendung pro Person -> Verbesserung des Ergebnisses für ein und dieselbe Person durch Selbstlernen während wiederholter Sitzungen
2.) Überwachung und Optimierung der Anwendung pro Einsatzzweck -> Verbesserung des Ergebnisses für ein bestimmtes Zustandsbild durch Selbstlernen, abgeleitet aus einer Gruppe von Personen während wiederholter Sitzungen
3.) Überwachung und Optimierung der Anwendung im kreativen Sinn und Verbesserung des Ergebnisses im Sinne der kreativen Neuheit durch Selbstlernen, abgeleitet aus der Gesamtheit der Sitzungen. Die im POOL Musik vorgehaltenen akustischen Signalabfolgen werden dabei nach und nach durch völlige Neu- und Eigenkreationen der KI ersetzt, sodass schlussendlich das System völlig selbständig neu kreiert und musiziert, ohne vorgefertigte Teilstücke zusammenzusetzen. Die Musik wird von der KI somit ab einem bestimmten Zeitpunkt de novo generiert, womit ab diesem Zeitpunkt der POOL Logik (9) und der POOL Musik (11) nur mehr der Erweiterung des Systems dienen.
4.) Optimierung der Gestenerkennung, z.B. Verbesserung des Ergebnisses durch Selbstlernen, sowohl für eine Person wie auch zugleich die Gesamtheit der Personen wie deren spezifischen Verhaltens- und Zustandsmuster.

Funktionsweise eines erfindungsgemäßen ersten Ausführungsbeispiels:
Die vom Atemsensor kommenden Daten werden in Bezug auf die Parameter Atemfrequenz, Atemperiode, Atemhub und Verhältnis von Einatmung:Ausatmung (I:E) analysiert. Je nach Wert von I:E wählt der SELEKTOR (8) eine Phrase aus, die im POOL MUSIK (11) kategorisiert für die I:E Verhältnisse 1:1, 1:2 und 1:3 in Form der Kategorien "EQUAL", "LONGER" und "OPTIMUM" vorliegen. Pro Sitzung sieht das System rund 10 Minuten vor. Diese Dauer von 600s wird im (annähernd) goldenen Schnitt berechnet in die zeitlichen Teilabschnitte, i.e. musikalischen Formteile, "INTRO" (96s), "STIM1" (228s), "STIM2" (144s) und "END" (132s) unterteilt. Im Bereich "INTRO" sind akustische Signalabfolgen aus den Kategorien "EQUAL" und "LONGER" verfügbar, in den Bereichen "STIM1" und "STIM2" aus allen drei Kategorien, im Schlussteil "END" stehen jedoch nur mehr die Kategorien "LONGER" und "OPTIMUM" zu Verfügung. Auf diese Weise und den im Folgenden beschriebenen Ablauf wird während der Sitzungszeit ein musikalischer Trend hin zu 1:3 generiert, welcher zugleich das Sitzungsziel - eine Verlängerung der Ausatmung, die eine Resonanzbildung zwischen Musik und Physiologie repräsentiert - realisiert.

### Ablauf:

**Phase 1:** Es werden Files aus dem Ordner "INTRO" gewählt, die einem I:E von 1:1, bzw. 1:2 im Wechsel entsprechen. Die Aufmerksamkeit wird auf den Melodiebau hingelenkt: Es soll erkenntlich werden, dass melodisches Geschehen die Atmung codiert.

**Phase 2 Unterabschnitt 1:** Es werden Files aus dem Ordner "STIM1" gewählt, die einem I:E von 1:2 entsprechen. Folgt die Person "spielerisch", oder befindet sie sich bereits am Beginn der Phase 2 auf diesem Niveau, werden Files aus dem Ordner "STIM1" gewählt, die 1:3 entsprechen. Bleibt es bei einem 1:2 Verhältnis, werden als Stimulation einzelne 1:3 Files "eingestreut".

**Phase 2 Unterabschnitt** 2: Es werden Files aus dem Ordner "STIM2" gewählt, die einem I:E von 1:3 entsprechen. Ergeben die Sensordaten, dass die Person nicht folgt/folgen kann, wird auf Einzelfiles aus "STIM2" mit dem Verhältnis 1:2 zurückgeschaltet und hernach sofort wieder auf 1:3 erhöht.

**Phase 3:** Es werden Files aus dem Ordner "END" gewählt, die wechselnd einem Verhältnis von 1:2 bzw. 1:3 entsprechen, je nach Sensordaten (mit Fokus auf 1:3).

Die dabei jeweils herrschende musikalische Ereignisdichte wird zudem durch die energetische Hüllkurve mitbestimmt.

Die ermittelte Atemfrequenz geht direkt in die Tempoinformation der Musik, der Atemhub in die Klangfarbeninformation (Filter, LFO, ...) ein, wobei die Zuordnungen variabel sind und über eine Matrix, MIDI-MATRIX (6) gewählt werden. Das musikalische Herzstück liegt dabei in der Codierung von Atemaktivität im Sinne funktioneller musikalischer Gestaltung. Die physiologischen Werte werden dazu v.a., jedoch nicht ausschließlich, in die musikalischen Parameter Tonfolgedichte und Tonhöhe (Ambitus) umgesetzt ("Ambitus-Spannung" und "melismatische Aktivität"). Das Verfahren kann auch "adaptomorphe Musik" bzw. "adaptomorphe Komposition" genannt werden. Die Gesamtdauer von 10 Minuten ist flexibel und richtet sich nach den Tempomodifikationen, die während der Sitzungsdauer erforderlich waren. Das System spielt dabei die jeweils letzte akustische Signalabfolge im Abschnitt "END" bis zur letzten "Note off" Information, sodass das Stück nicht abbricht.

Schutzmechanismus: Wird eine Atemfrequenz von unter 6/min oder über 35/min erhoben, schaltet das System ab.

Erweiterung um gestengetriggerte (bewegungsgetriggerte) Phrasen: Durch ein Hinzunehmen des Bewegungssensors kann die adaptomorphe Musik mehrstimmig, kontrapunktisch und rhythmisch-metrisch strukturiert und gesteuert werden, indem die benutzende Person via Gesten Phrasen triggert (siehe unten), womit hoch effektive Entrainment Effekte genützt werden können (Haas, François & Distenfeld, S & Axen, K. (1986). Effects of perceived musical rhythm on respiratory pattern. Journal of applied physiology (Bethesda, Md.: 1985). 61. 1185-91. 10.1152/jappl.1986.61.3.1185.).

Gemäß einer zweiten Ausführungsform werden die vom Bewegungssensor kommenden Daten analysiert und als spezifische sowie relevante, in Form einer Datenbank vorgehaltener, PG=POOL GESTEN (5), Gesten, d.h. Bewegungen, erkannt. Dabei wird zugleich die Qualität der Ausführung beurteilt, indem die Geschwindigkeit der Geste und der Grad der Vollendung der Geste ermittelt werden. Zudem wird ermittelt, ob einhändig oder beidhändig agiert wird, welche Hand aktiv ist und ob bei beidhändiger Ausführung simultan oder sequentiell vorgegangen wird. Je nach erfolgreich ausgeführter Geste, wird vom SELEKTOR (8) eine Phrase aus dem POOL MUSIK (11) gewählt und über den weiter oben bereits beschriebenen Weg als hörbares musikalisches Ereignis ausgegeben.

Eine Trainingsphase umfasst etwa 20 Minuten.

Diese teilt sich in FÜNF PHASEN zu je 4 Minuten Dauer. Sollte die Person eine Phase bereits "spielerisch" beherrschen, kann diese Phase übersprungen/verkürzt werden, die jeweilige Restdauer wird den nachfolgenden Abschnitten zu gleichen Teilen zugeschlagen, bei weiterem Überspringen/Verkürzen verbleibt die diesbezüglich aufsummierte Restzeit somit zur Gänze bei der letzten Phase (Beispiel I: Phase 1 nur 2 Minuten benötigt, daher erhöht sich die Dauer der nachfolgenden Einheiten auf je 4 Minuten und 30 Sekunden. Beispiel II: Phase I - IV werden in je 2 Minuten absolviert, daher verbleiben für Phase V 12 Minuten.)

**PHASE 1: "HEBEN"** Gesten/Bewegungen: Flexion und Extension (Ellbogengelenk).

Ablauf: Es werden Files aus dem Ordner "HEBEN" gewählt, die aneinandergereiht durchspielen. Die Distanz zum Gestencontroller wirkt auf diese Files hinsichtlich der Steuerung von KLANGFARBE (TIMBRE) und VIBRATO ein.

**PHASE 2: "DREHEN"** Gesten/Bewegungen: Pronation und Supination (Unterarm).

Ablauf: Es werden Files aus dem Ordner "DREHEN" gewählt, die aneinandergereiht durchspielen. Die Abweichung des Drehwinkels der Hand von der Lotrechten auf das Sensorfeld wirkt auf diese Files hinsichtlich der Steuerung von: KLANGFARBE (TIMBRE), PITCH BEND und VIBRATO ein.

**PHASE 3: "FAUST"** Gesten/Bewegungen: (großer) Faustschluss (Flexion Fingergelenke bzw. Opposition Daumen).

Ablauf: Es werden Files aus dem Ordner "FAUST" gewählt, die aneinandergereiht durchspielen. Die vollständig geöffnete oder geschlossene Hand (als eindeutige Geste) wirkt auf diese Files hinsichtlich ihrer Auswahl (kategorisiert in den Unterordnern "FOPEN" und "FCLOSED") ein und des Weiteren auf die Steuerung von KLANGFARBE (TIMBRE), VIBRATO und LAUSTÄRKE (diesbezüglich gilt als Standard DEFAULT: geschlossene Faust = STILLE - dies kann auch invertiert werden).

**PHASE 4: "KLOPFEN"** Gesten/Bewegungen: "Klopfbewegungen" (Palmarflexion und Dorsalextension im Handgelenk).

Ablauf: Es werden Files aus dem Ordner "KLOPFEN" gewählt, die aneinandergereiht durchspielen. Die Bewegungsrichtung der Hand wirkt auf diese Files hinsichtlich ihres TEMPOS ein. Dazu können einzelne Töne/Sounds durch die entsprechende Klopfbewegung direkt getriggert werden ("Schlagzeugfunktion"). Als Triggerpunkt für den Sensor werden ausschließlich die Endpunkte der Palmarflexion verwendet.

**PHASE 5: "FINGER"** Gesten/Bewegungen: Zueinanderführen der jeweiligen Fingerkuppen (Fingerschluss) beider Hände in den Kombinationen Daumen-Zeigefinger | Daumen-Mittelfinger | Daumen-Ringfinger | Daumen-Kleiner Finger.

Ablauf: Es werden Files aus dem Ordner "FINGER" gewählt. Die Nummerierung der Bewegungen lautet dabei systemintern immer wie folgt: 1 Daumen - Zeigefinger RECHTS | 2 Daumen - Zeigefinger LINKS | 3 Daumen - Mittelfinger RECHTS | 4 Daumen - Mittelfinger LINKS | 5 Daumen - Ringfinger RECHTS | 6 Daumen - Ringfinger LINKS | 7 Daumen - Kleiner Finger RECHTS | 8 Daumen - Kleiner Finger LINKS. Bei jedem erfolgten Fingerschluss (mit Toleranzbreite siehe unten) wird das eineindeutig 1-8 zugeordnete File gestartet. Für die Therapeut/innen stehen Zusatzfiles für den Zweispielermodus zur Verfügung. Diese werden über einen zweiten Controller ausgelöst und sind ebenfalls den einzelnen Fingerschlüssen 1-8 durchnummeriert zugeordnet. In der Gesamtheit entstehen im Zusammenspiel Musikstücke mit Begleitung. Ergänzend können die Files in ihrer Klangfarbe beeinflusst werden. Zusatzfiles ermöglichen Variationsbildung, die Files werden dabei rein algorithmisch getriggert: Jeder zweite Übungsdurchlauf erhält eine Variation. Zusatzfunktion: Die Einzelübungen werden durch Überleitungsfiles, die ebenfalls rein algorithmisch ausgelöst werden, verbunden. Jede Übung läuft fünfmal durch, die Wiederholungen können übersprungen werden. Der Gesamtablauf der PHASE 5 kann über ein Eingabefeld am GUI (1) im Tempo stufenlos reguliert werden. Die Realisierung des Fingerschlusses (Toleranzbreite) kann sensorseitig dreistufig in Bezug auf den Triggerpunkt voreingestellt werden: a.) vollkommen, b.) näherungsweise, c.) allfällig erkennbar.

Durch ein Hinzunehmen des Atemsensors kann die adaptomorphe Musik mehrstimmig strukturiert und gesteuert werden, indem die benutzende Person via Atmung Steuerbefehle übermittelt (v.a. MIDI-CC Controller) und damit die Klangfarbe beeinflusst oder Phrasen triggert.

Die Klangfarbe kann frei gewählt werden, die Stimmung/das Tonsystem kann frei gewählt werden. Im Prinzip kann auch das jeweilige VSTi Instrument frei gewählt werden.

### Beispiel 2:

### Schritt A

Unter Nutzung eines Sensors, wobei ein
a. 3D Infrarot Gestensensor
b. Atemsensor (in jeder erdenklichen Ausführung: Brustgurt-Zugsystem, RADAR, aus EKG-Sensordaten abgeleitete Atemkurven, vermittels dem 3D Infrarot Gestensensor willentlich generierte Triggersignale, usw.)
c. und Steuer- bzw. Regel-Daten von Beatmungsmaschinen (jedweden Fabrikats, Herstellers, Aufbaus und Funktionsmusters) zum Einsatz kommen, werden physiologische Daten, die für die Atmung und Bewegung signifikant sind, erfasst und hinsichtlich der Periodizität (wie insbesondere der Periodendauern) der darin auftretenden Werte (wie deren jeweiligen Trends) in Echtzeit analysiert. Im Zuge dessen werden Kategorien gebildet, die in weiterer Folge die Entscheidungslogik des Systems beeinflussen.

Auf diese Weise wird erhoben und im System festgelegt, ob etwa
a. das (zeitliche) Verhältnis von Ein- und Ausatmung (I:E) den Mustern 1:1, 1:2, 1:3, 1:4, 1:5, 1:6, 1:7, 1:8, 1:9, 1:10, 1:11, 1:12, 1:13, 1:14, 1:15 etc. entspricht
b. oder die Vollendung einer zyklisch repetitiven Bewegung der Hände (1 - vollendet, 0,9 ... 0,6 größtenteils vollendet, 0,5 halbvollendet, 0,4 ..... 0,1 teilvollendet, 0 komplett unvollendet) gegeben ist.

### Schritt B

Musikalische Phrasen werden komponiert. Das sind der allgemein üblichen Definition nach musikalische Teilabschnitte/Teilstücke (funktionell-kompositorische Bausteine), die zumindest, jedoch nicht ausschließlich, zwei Töne umfassen und damit eine bestimmte musikalische Zeitstrecke und darin einen musikalischen Verlauf (z.B. einen Melodiebogen) definieren.

Bei der Gestaltung dieser einzelnen Phrasen wird kompositorisch so vorgegangen, dass alle musikalischen Parameter, welche die Phrasen ausmachen, im Sinne funktioneller Musik auf die akustische Repräsentation und Triggerung von menschlicher (physiologischer) Atmung und Bewegung abgestimmt werden.

Dabei werden Kategorien gebildet, die sowohl den auch in Schritt A verwendeten physiologischen Kategorien (z.B. 1:1, 1:2, 1:3) wie auch unterschiedlichen musikalischen Energieniveaus (skaliert von 0-127, wobei 0 - minimale ENERGIE und 127 - maximale ENERGIE bedeutet) entsprechen.

Jede einzelne Phrase wird so konzipiert, dass sie sowohl bei zeitlich aufeinander folgender als auch simultaner Verwendung mit jeder anderen im System vorgehaltenen Phrase musikalisch sinnvoll kombiniert werden kann.

Ein System an Regeln wird definiert, welches diese sinnvolle, auf rein musikalischen Gesetzmäßigkeiten basierende Kombination der Phrasen untereinander festlegt. Die einzelnen Phrasen können dabei ein- oder mehrstimmig angelegt sein oder auch nur rhythmische Informationen beinhalten. Die Phrasen werden im Format MIDI (.mid) im System gespeichert, das Regelsystem in Form einer Datenbank.

### Schritt C

Ein musikalischer Formverlauf (= eine musikalische Form) wird definiert, indem dessen Zeitdauer (z.B. 3 Minuten, 5 Minuten, 10 Minuten, 20 Minuten) und Unterabschnitte (dem klinischen Einsatzfeld entsprechend benannt in INTRO - STIM 1...STIM x - END) bei jeder einzelnen Anwendung aufs Neue definiert werden, wobei für jeden zu bestimmenden Unterabschnitt derart ein Zeitwert definiert wird, sodass sich die Einzelsummen aller Unterabschnitte zu der vorab festgelegten Gesamtzeit summieren. (Fig. 4)

Für die Gesamtheit der musikalischen Form - wie für jeden Unterabschnitt - wird im Zuge dessen zugleich eine energetische Hüllkurve festgelegt. Die dabei zu bestimmenden Werte umfassen, jedoch nicht ausschließlich, zumindest den Zeit- und Amplitudenverlauf der Kurve, indem Breakpoints festgesetzt werden. Jeder Breakpoint ist durch einen Zeitwert und numerischen Wert definiert. Der Verlauf der Kurve zwischen den einzelnen Breakpoints ist dabei beliebig formbar. Die Anzahl der Breakpoints ist im Prinzip beliebig, der zeitliche Abstand der Breakpoints ebenso, jedoch ist dieser in Bezug auf den dabei kürzest möglichen Abstand mit der Länge der kürzesten im System vorgehaltenen, in Schritt B vorproduzierten musikalischen Phrase korreliert. (Fig. 5)

### Schritt D

Ein Algorithmus (genannt: SELEKTOR) wird definiert, der
a. auf Basis der in Schritt A ermittelten Kategorien und
b. unter Berücksichtigung der in Schritt C definierten Formabschnitte und energetischen Hüllkurve im Schritt B erstellte musikalische Phrasen auswählt und solchermaßen auf Basis des musikalischen Regelwerkes sinnvoll aneinanderfügt, dass sie einen musikalischen Gesamtverlauf ergeben, der
c. die jeweils aktuellen physiologischen IST-Situationen im Sitzungsverlauf
d. den gewünschten, vor jeder Sitzung festgelegten musikalischen Formverlauf (wie dessen Substruktur)
e. den gewünschten, vor jeder Sitzung festgelegten musikalischen Energieverlauf und
f. den gewünschten, allgemein festgelegten physiologischen Trendverlauf hin zu Normwerten von Atmung und Bewegung exakt repräsentiert und dabei von hohem ästhetischem Wert und klinisch wirksam ist.

Der Algorithmus passt dabei zugleich, jedoch nicht ausschließlich, die jeweilige Geschwindigkeit, aber z.B. auch Klangfarbe, Lautstärke, Transposition bzw. Tonhöhe, der Phrasen solchermaßen aneinander an, dass die vorausbestimmte Spieldauer der festgelegten Gesamtform nicht um mehr als 10% über- oder unterschritten wird und sich der Übergang von einer in die nächste Phrase unmerklich gestaltet, zudem wird das Tempo zu jedem Zeitpunkt mit der Periodendauer der physiologischen Signale korreliert.

### SCHRITT E

Der solchermaßen generierte Datenfluss wird in Echtzeit als Steuerdaten (im Format MIDI .mid) ausgegeben, bzw. als Musikfile (MIDI-File) im System gespeichert. Über virtuelle MIDI-Kabel werden diese MIDI-Daten innerhalb des Systems an ein Software-Instrument (Software-Sampler, Software-Synthesizer, z.B. im Schnittstellenformat VSTi) weitergeleitet (bzw. via einem MIDI-Hardware-Interface nach außen an Hardwareklangerzeuger weitergegeben) und durch diese Software- bzw. Hardwareinstrumente in Audiosignale beliebiger Klangfarbe etc. übersetzt und via Lautsprecher/Kopfhörer beliebiger Bauart abgestrahlt.

Im Zuge der klinischen Verwendung ist dabei auf die Einhaltung von gültigen Sicherheitsnormen/EU-Normen in Bezug auf die maximal abgegebene Lautstärke zu achten. Des Weiteren ist darauf zu achten, dass Personen durch die akustischen Signale nicht erschrecken können.

Figur 6: Flussdiagramm der Schritte A bis E und darin bestehende Abhängigkeiten in Bezug auf den Selektor.

### Beispiel 3

Das Beispiel beschreibt eine Therapiesitzung mit einer Dauer von 10 Minuten mit einem Patienten auf einer Intensivstation. Der Kurvenverlauf a) Atemperiode, b) Atemhub, c) Verhältnis Ein-Ausatmung zeigt deutlich eine entsprechende physiologische Reaktion in dem von der Anwendung intendierten Sinne. Dabei sind Regulationsmuster ersichtlich, die sich in dynamischer Reaktion auf das musikalische Geschehen als eine Verlängerung der Atemperiode (Kurve a)) und "gezieltem Atemfluss" folgenden Trends in Bezug auf das Verhältnis von Ein- zu Ausatmung I:E (Kurve b)) bei gleichzeitiger Stabilisierung des Atemhubes (Kurve c)) darstellen.

Die physiologischen Daten zeigt Figur 7.

Das I:E-Verhältnis erzielt unter Musikeinfluss dabei einen Gipfelwert während der aufgezeichneten Messperiode (Pfeil in 7b)). Die Daten wurden mittels des Atemsensors (auf Zuggurtbasis) einer Vorrichtung gemäß der Erfindung erhoben; die Datenspeicherung der einzelnen Sitzungen erfolgt im System routinemäßig. Ergänzend wurden auf der Intensivstation die ebenfalls routinemäßig ins Patientendatenmanagementsystem einmündenden Daten wie Blutdruck, Herzrate, usw. dokumentiert und zudem ein musiktherapeutisches Protokoll (inklusive einer Befragung zur Hör-Biographie) verfasst. Dabei zeigen sich u.a. Reaktionen wie eine Senkung des Blutdrucks bzw. der Herzrate. Bei dem im Beispiel gezeigten Patienten, der an drei hintereinander folgenden Tagen eine Sitzung mit der erfindungsgemäßen Vorrichtung absolvierte, ergaben sich dabei bereits während der Sitzung am Tag 2 folgende Blutdruckwerte: Bei Start der Anwendung: 159/96, nach 5 Minuten Laufzeit 143/74, nach 10 Minuten Laufzeit bei Ende der Anwendung 138/77 und 30 Minuten NACH erfolgter Anwendung 137/77. Dies spricht, zumal sich der Trend 30 Minuten nach der Sitzung als stabil erwies, für eine deutlich positive Reaktion des Patienten auf die Anwendung. Es erfolgten positive verbale Reaktionen des Patienten auf die einzelnen Sitzungen, wobei der Patient vor der Sitzung drei auch Wünsche im Hinblick auf die Klangfarbe äußerte und sich für die Umsetzung selbiger im Anschluss bedankte.

## Patentansprüche

1. Verfahren zur Ausgabe eines akustischen Signals, welches aus akustischen Signalabfolgen bestehend aus aneinandergereihten akustischen Sequenzen von 0,5 bis 70 Sekunden, und/oder Teilsignalen aufgebaut ist und wobei der Aufbau des akustischen Signals auf physiologischen Daten beruht, **dadurch gekennzeichnet, dass** es folgende Verfahrensschritte umfasst:
- ausgewählte sensorermittelte physiologische Daten einer Person, umfassend zumindest das Verhältnis von Ein- und Ausatmungsdauer werden bereitgestellt,
- der Verlauf von Parametern der physiologischen Daten, ausgewählt aus dem Verhältnis von Ein- und Ausatmung, Atemfrequenz, Atemperiode, Atemhub, Bewegungen der Finger, Hände und Arme und die zeitlichen Trends dieser Parameter werden diskretisiert, quantisiert und kategorisiert indem bei der Kategorisierung die Teilsignale und/oder die Signalabfolgen einem Verhältnis der physiologischen Parameter, einer energetischen Hüllkurve und musikalischen Teilabschnitten zugeordnet werden,
- den so erhaltenen Kategorien der Parameter werden unter Einbeziehung von mindestens vier musikalischen Formteilen, welche strukturbildende Unterabschnitte eines Musikwerks sind, und einer energetischen Hüllkurve akustische Signalabfolgen zugeordnet,
- die akustischen Signalabfolgen werden entsprechend dem Verlauf der kategorisierten Parameter der physiologischen Daten in Echtzeit zu einem finalen akustischen Signal kombiniert und ausgegeben, wobei
die Kombination und Ausgabe der akustischen Signalabfolgen zu einer akustischen Gesamtsequenz nach einer Verknüpfungslogik erfolgt, welche den musikalischen Gesetzmäßigkeiten der europäischen und/oder außereuropäischen Kunstmusik folgt, ausgewählt aus der Abfolge von Tonarten und den darin festgesetzten Tonstufen, Abfolge von melodischen und harmonischen Fortschreitungen, Abfolge von musikalischen Formteilen, Abfolge von Klangfarben, Rhythmen und Tempi, wobei diese Gesetzmäßigkeiten in einer Datenbank enthalten sind, und das akustische Signal zu einem Klangbild geformt wird, das in seinem zeitlichen Verlauf jedes Mal neu und einmalig entsteht, und
die Zuordnung der akustischen Signalabfolgen zu den Kategorien gemäß einer Bildungsvorschrift erfolgt, dass zumindest vier Teilabschnitte eines festgelegten Zeitverlaufs von 10 bis 20 Minuten unter Berücksichtigung des Setzens von Stimuli in Richtung von physiologisch normvarianten Ziel- und Sollwerten und unter Berücksichtigung musikalischer Regeln derart mit dem jeweiligen physiologischen Zustand korrelierten akustischen Signalfolgen ausgefüllt werden, dass sich eine kontinuierliche und jeweils neuartige akustische Gesamtsequenz ergibt.

2. Verfahren nach Anspruch 1, **dadurch gekennzeichnet, dass** bei physiologischen Daten mit einem periodischen Verlauf die Periodendauer und/oder der Amplitudenverlauf als Parameter herangezogen wird.

3. Verfahren nach Anspruch 1 oder 2, **dadurch gekennzeichnet, dass** eine Speichereinheit mit einer Datenbank enthaltend eine Vielzahl akustischer Teilsignale und/oder Signalabfolgen verwendet wird.

4. Verfahren nach einem der Ansprüche 1 bis 3, **dadurch gekennzeichnet, dass** die ausgewählten sensorermittelten physiologischen Daten einer Person zusätzlich die Atemfrequenz, die Atemperiode und/oder den Atemhub umfassen.

5. Verfahren nach einem der Ansprüche 1 bis 4, **dadurch gekennzeichnet, dass** die ausgewählten sensorermittelten physiologischen Daten durch einen Atemsensor, eine Kamera, einen Bewegungssensor, oder einen 3D-Infrarot-Gestencontroller bereitgestellt werden, und optional zusätzlich ein Sensor zur Aufnahme der Herzfrequenz verwendet wird.

6. Verfahren nach einem der Ansprüche 1 bis 5 **dadurch gekennzeichnet, dass** die ausgewählten sensorermittelten physiologischen Daten durch Vorrichtungen zur maschinellen Beatmung und zum Monitoring von Patientendaten bereitgestellt werden.

7. Verfahren nach einem der Ansprüche 1 bis 6, **dadurch gekennzeichnet, dass** die akustischen Teilsignale und/oder Signalabfolgen zu einer Vielzahl von verschiedenen Sequenzen verbindbar sind, die das akustische Signal bilden, wobei insbesondere die Sequenzen eine Variation hinsichtlich der Signalabfolgen, der Tonfolgedichte, der Tonhöhe, der Stimmenzahl, der Lautstärke, der Artikulation, der Klangfarbe, des Tempos und/oder des Rhythmus aufweisen.

8. Verfahren nach einem der Ansprüche 1 bis 7, **dadurch gekennzeichnet, dass** die akustischen Teilsignale und/oder Signalabfolgen zu einer Vielzahl von verschiedenen Sequenzen verbindbar sind und die akustischen Teilsignale und/oder Signalabfolgen eine Variation hinsichtlich ihrer Klangattribute, insbesondere Klangart, Klangfarbe, Tonhöhe, Artikulation und/oder Lautstärke aufweisen.

9. Verfahren nach einem der Ansprüche 1 bis 8, **dadurch gekennzeichnet, dass** ausgewählte sensorermittelte physiologische Daten einer Person bereitgestellt werden, umfassend Bewegungen einer oder beider Hände und/oder Arme, insbesondere umfassend die Geschwindigkeit und den Grad der Vollendung dieser Bewegungen.

10. Vorrichtung angepasst für die Durchführung des Verfahrens nach einem der Ansprüche 1 bis 9, **dadurch gekennzeichnet, dass** die Vorrichtung zumindest folgendes enthält:
- zumindest einen Sensor (2) zur Erfassung ausgewählter physiologischer Daten einer Person, umfassend das Verhältnis von Ein- und Ausatmungsdauer (I:E) und/oder die Bewegungen der Hände,
- STAT-Modul (3) zur Diskretisierung, Quantisierung und Kategorisierung des Verlaufs vorgegebener Parameter der physiologischen Daten,
- SELEKTOR (8) zur Zuordnung akustischer Signalabfolgen zu den Kategorien und algorithmischen Komposition entsprechend dem Verlauf der kategorisierten Parameter der physiologischen Daten, und
- INTEGRATOR (10) zur kontinuierlichen Kombination der akustischen Signalabfolgen zu einem akustischen Signal und zur Ausgabe des Signals, und die akustischen Teilsignale und/oder Signalabfolgen in einer Datenbank gespeichert sind und in zumindest 4 Kategorien eingeteilt sind:
a. Kategorie 1 umfassend Teilsignale und/oder Signalabfolgen, die einem Verhältnis der Dauer von Ein- und Ausatmung von 1:1 entsprechen;
b. Kategorie 2 umfassend Teilsignale und/oder Signalabfolgen, die einem Verhältnis der Dauer von Ein- und Ausatmung von 1:2 entsprechen;
c. Kategorie 3 umfassend Teilsignale und/oder Signalabfolgen, die einem Verhältnis der Dauer von Ein- und Ausatmung von 1:3 entsprechen;
d. Kategorie 4 umfassend Teilsignale und/oder Signalabfolgen, die bestimmten Bewegungen der Finger, Hände und Arme entsprechen.

11. Vorrichtung nach Anspruch 10, **dadurch gekennzeichnet, dass** die Kategorien 1 bis 3 in die Abschnitte "INTRO", "STIM1", "STIM2", "END" unterteilt sind, und Kategorie 4 einem oder mehreren Abschnitten zugeordnet ist, die bevorzugt nach Fingern, Händen und Armen benannt sind.

12. Vorrichtung nach Anspruch 11, **dadurch gekennzeichnet, dass** die Regeln der musikalischen Verknüpfungslogik für die einzelnen akustischen Teilsignale und/oder Signalabfolgen sowie für die Abfolge der einzelnen Zeitabschnitte und die energetische Hüllkurve in einer Datenbank gespeichert sind.

13. Computerimplementiertes Verfahren zur Zuordnung physiologischer Daten zu mindestens einem akustischen Teilsignal oder einer akustischen Signalabfolge und zur Erstellung einer Klangabfolge zusammengesetzt aus mindestens zwei akustischen Signalen nach einem Verfahren der Ansprüche 3 bis 9, umfassend die Schritte:
a. Bereitstellen einer Datenbank umfassend eine Vielzahl akustischer Teilsignale und/oder Signalabfolgen;
b. Ermittlung physiologischer Daten zumindest mittels einem Atemsensor oder einem Bewegungssensor, wobei die physiologischen Daten mindestens das Verhältnis von Ein- und Ausatmungsdauer umfassen;
c. Auswerten der physiologischen Daten;
d. Zuordnen der physiologischen Daten zu einem akustischen Teilsignal oder einer akustischen Signalabfolge;
e. Ergänzendes Zuordnen der akustischen Teilsignale und/oder Signalabfolgen zu zumindest vier musikalischen Formteilen und einer energetischen Hüllkurve; und
f. Erstellung und Ausgabe einer Klangabfolge zusammengesetzt aus mindestens zwei akustischen Teilsignalen, Signalabfolgen, oder Signalen.

14. Verwendung des Verfahrens nach einem der Ansprüche 1 bis 9, angepasst für die Modulation der Atemfrequenz, Atemperiode und/oder des Verhältnisses von Ein- und Ausatmungsdauer oder zum Training der Koordination von Handbewegungen.

15. Verfahren nach einem der Ansprüche 1 bis 9, angepasst für die Modulation des Atemmusters einer Person, umfassend die Schritte:
a. Bereitstellen einer Datenbank umfassend eine Vielzahl akustischer Teilsignale und/oder Signalabfolgen;
b. Ermittlung physiologischer Daten der Person mit zumindest einem Atemsensor und vorzugsweise einem Bewegungssensor, wobei die physiologischen Daten mindestens das Verhältnis von Ein- und Ausatmungsdauer, und optional die Atemfrequenz, Atemperiode und/oder den Atemhub und spezifische Bewegungen der Finger, Hände, oder Arme, und die Geschwindigkeit und den Grad der Vollendung der Bewegungen einer oder beider Hände und/oder Arme umfassen;
c. Auswerten der physiologischen Daten;
d. Zuordnen der physiologischen Daten zu einem akustischen Teilsignal oder einer akustischen Signalabfolge;
e. Zuordnen der akustischen Teilsignale und/oder Signalabfolgen zu zumindest vier musikalischen Formteilen und einer energetischen Hüllkurve;
f. Erstellung einer Klangabfolge zusammengesetzt aus mindestens zwei akustischen Teilsignalen oder Signalabfolgen; und
g. Ausgabe der Klangabfolge an die Person,
wobei die Kombination und Ausgabe der akustischen Signalabfolgen zu einer akustischen Gesamtsequenz nach einer Verknüpfungslogik erfolgt, welche den musikalischen Gesetzmäßigkeiten der europäischen und/oder außereuropäischen Kunstmusik folgt, ausgewählt aus der Abfolge von Tonarten und den darin festgesetzten Tonstufen, Abfolge von melodischen und harmonischen Fortschreitungen, Abfolge von musikalischen Formteilen, Abfolge von Klangfarben, Rhythmen und Tempi, wobei diese Gesetzmäßigkeiten in einer Datenbank enthalten sind, und das akustische Signal zu einem Klangbild geformt wird, das in seinem zeitlichen Verlauf jedes Mal neu und einmalig entsteht.

16. Verfahren nach Anspruch 14, wobei die Modulation des Atemmusters die Verlängerung der Ausatmung im Verhältnis zur Einatmung umfasst.

17. Verfahren zum Training der Handkoordination einer Person nach einem der Verfahren von 1 bis 9, umfassend die Schritte:
a. Bereitstellen einer Datenbank umfassend eine Vielzahl akustischer Teilsignale und/oder Signalabfolgen;
b. Bereitstellung physiologischer Daten der Person durch einen Bewegungssensor und optional einen Atemsensor, wobei die physiologischen Daten die Geschwindigkeit und Vollendung der Bewegungen einer oder beider Hände und/oder Arme umfassen, und das Verhältnis von Ein- und Ausatmungsdauer, die Atemfrequenz, Atemperiode und/oder den Atemhub;
c. Auswerten der physiologischen Daten;
d. Zuordnen der physiologischen Daten zu einem akustischen Teilsignal oder einer akustischen Signalabfolge;
e. Erstellung einer Klangabfolge zusammengesetzt aus mindestens zwei akustischen Teilsignalen oder Signalabfolgen; und
f. Ausgabe der Klangabfolge an die Person,
wobei die Kombination und Ausgabe der akustischen Signalabfolgen zu einer akustischen Gesamtsequenz nach einer Verknüpfungslogik erfolgt, welche den musikalischen Gesetzmäßigkeiten der europäischen und/oder außereuropäischen Kunstmusik folgt, ausgewählt aus der Abfolge von Tonarten und den darin festgesetzten Tonstufen, Abfolge von melodischen und harmonischen Fortschreitungen, Abfolge von musikalischen Formteilen, Abfolge von Klangfarben, Rhythmen und Tempi, wobei diese Gesetzmäßigkeiten in einer Datenbank enthalten sind, und das akustische Signal zu einem Klangbild geformt wird, das in seinem zeitlichen Verlauf jedes Mal neu und einmalig entsteht.

## Claims

1. A method for outputting an acoustic signal that is formed from acoustic signal series consisting of concatenated acoustic sequences of 0.5 to 70 seconds, and/or from part-signals, and wherein the structure of the acoustic signal is based on physiological data, **characterised in that** the method comprises the following method steps:
- selected sensor-determined physiological data of a person, comprising at least the ratio of inspiration duration to expiration duration, are provided,
- the profile of parameters of the physiological data, selected from the ratio of inspiration duration to expiration duration, respiratory frequency, respiratory period, tidal volume, movements of the fingers, hands and arms, and the trends of these parameters over time are discretised, quantised and categorised by assigning, in the categorisation, the part-signals and/or the signal series to a ratio of the physiological parameters, to an energetic envelope, and to musical part-sections,
- the categories thus obtained of the parameters are assigned acoustic signal series, including at least four musical form elements, which are structure-forming sub-sections of a musical work, and an energetic envelope,
- the acoustic signal series are combined and output according to the profile of the categorised parameters of the physiological data in real time to form a final acoustic signal, wherein
the combination and output of the acoustic signal series to form an overall acoustic sequence takes place according to a connective logic that follows the musical principles of European and/or non-European art music, selected from the series of keys and the degrees defined therein, series of melodic and harmonic progressions, series of musical form elements, series of timbres, rhythms and tempos, wherein these principles are present in a database, and the acoustic signal is formed into an acoustic pattern that develops anew and uniquely each time in its chronological profile, and
the assignment of the acoustic signal series to the categories takes place according to a formation rule that at least four part-sections of a defined time profile of 10 to 20 minutes are filled with acoustic signal series that are correlated with the particular physiological state, taking into account the setting of stimuli in the direction of physiologically norm-variant target and setpoint values and taking into account musical rules, such that a continuous and in each case novel overall acoustic sequence develops.

2. The method according to Claim 1, **characterised in that**, in the case of physiological data having a periodic profile, the period duration and/or the amplitude profile is used as a parameter.

3. The method according to Claim 1 or 2, **characterised in that** a memory unit having a database containing a plurality of acoustic part-signals and/or signal series is used.

4. The method according to one of Claims 1 to 3, **characterised in that** the selected sensor-determined physiological data of a person additionally comprise the respiratory frequency, the respiratory period and/or the tidal volume.

5. The method according to one of Claims 1 to 4, **characterised in that** the selected sensor-determined physiological data are provided by a respiratory sensor, a camera, a movement sensor, or a 3D infrared gesture controller, and a sensor for detecting the heart rate is optionally additionally used.

6. The method according to one of Claims 1 to 5, **characterised in that** the selected sensor-determined physiological data are provided by devices for mechanical ventilation and for monitoring patient data.

7. The method according to one of Claims 1 to 6, **characterised in that** the acoustic part-signals and/or signal series can be linked to form a plurality of different sequences that form the acoustic signal, wherein in particular the sequences vary in terms of the signal series, melodic density, pitch, number of voices, loudness, articulation, timbre, tempo and/or rhythm.

8. The method according to one of Claims 1 to 7, **characterised in that** the acoustic part-signals and/or signal series can be linked to form a plurality of different sequences, and the acoustic part-signals and/or signal series vary in terms of their tonal attributes, in particular tone quality, timbre, pitch, articulation and/or loudness.

9. The method according to one of Claims 1 to 8, **characterised in that** selected sensor-determined physiological data of a person are provided, comprising movements of one or both hands and/or arms, in particular comprising the speed and degree of completion of these movements.

10. A device adapted for carrying out the method according to one of Claims 1 to 9, **characterised in that** the device contains at least the following:
- at least one sensor (2) for detecting selected physiological data of a person, comprising the ratio of inspiration duration to expiration duration (I:E) and/or the movements of the hands,
- STAT module (3) for discretising, quantising and categorising the profile of specified parameters of the physiological data,
- SELECTOR (8) for assigning acoustic signal series to the categories and algorithmic composition according to the profile of the categorised parameters of the physiological data, and
- INTEGRATOR (10) for continuously combining the acoustic signal series to form an acoustic signal and for outputting the signal, and the acoustic part-signals and/or signal series are stored in a database and classified into at least 4 categories:
a. category 1 comprising part-signals and/or signal series that correspond to a ratio of inspiration duration to expiration duration of 1:1;
b. category 2 comprising part-signals and/or signal series that correspond to a ratio of inspiration duration to expiration duration of 1:2;
c. category 3 comprising part-signals and/or signal series that correspond to a ratio of inspiration duration to expiration duration of 1:3;
d. category 4 comprising part-signals and/or signal series that correspond to certain movements of the fingers, hands and arms.

11. The device according to Claim 10, **characterised in that** categories 1 to 3 are divided into the sections "INTRO", "STIM1", "STIM2", "END", and category 4 is assigned to one or more sections that are preferably named after fingers, hands and arms.

12. The device according to Claim 11, **characterised in that** the rules of the musical connective logic for the individual acoustic part-signals and/or signal series and for the series of individual time sections and the energetic envelope are stored in a database.

13. A computer-implemented method for assigning physiological data to at least one acoustic part-signal or acoustic signal series and for creating a series of sounds composed of at least two acoustic signals by a method of Claims 3 to 9, comprising the steps of:
a. providing a database comprising a plurality of acoustic part-signals and/or signal series;
b. determining physiological data at least by means of a respiratory sensor or a movement sensor, wherein the physiological data comprise at least the ratio of inspiration duration to expiration duration;
c. evaluating the physiological data;
d. assigning the physiological data to an acoustic part-signal or an acoustic signal series;
e. additionally assigning the acoustic part-signals and/or signal series to at least four musical form elements and an energetic envelope; and
f. creating and outputting a series of sounds composed of at least two acoustic part-signals, signal series, or signals.

14. The use of the method according to one of Claims 1 to 9, adapted for the modulation of the respiratory frequency, respiratory period and/or the ratio of inspiration duration to expiration duration or for training the coordination of hand movements.

15. The method according to one of Claims 1 to 9, adapted for the modulation of the breathing pattern of a person, comprising the steps of:
a. providing a database comprising a plurality of acoustic part-signals and/or signal series;
b. determining physiological data of the person using at least one respiratory sensor and preferably a movement sensor, wherein the physiological data comprise at least the ratio of inspiration duration to expiration duration, and optionally the respiratory frequency, respiratory period and/or tidal volume and specific movements of the fingers, hands or arms, and the speed and degree of completion of the movements of one or both hands and/or arms;
c. evaluating the physiological data;
d. assigning the physiological data to an acoustic part-signal or an acoustic signal series;
e. assigning the acoustic part-signals and/or signal series to at least four musical form elements and an energetic envelope;
f. creating a series of sounds composed of at least two acoustic part-signals or signal series; and
g. outputting the series of sounds to the person,
wherein the combination and output of the acoustic signal series to form an overall acoustic sequence takes place according to a connective logic that follows the musical principles of European and/or non-European art music, selected from the series of keys and the degrees defined therein, series of melodic and harmonic progressions, series of musical form elements, series of timbres, rhythms and tempos, wherein these principles are present in a database, and the acoustic signal is formed into an acoustic pattern that develops anew and uniquely each time in its chronological profile.

16. The method according to Claim 14, wherein the modulation of the breathing pattern comprises lengthening expiration in relation to inspiration.

17. A method for training the hand coordination of a person by one of the methods of 1 to 9, comprising the steps of:
a. providing a database comprising a plurality of acoustic part-signals and/or signal series;
b. providing physiological data of the person by means of a movement sensor and optionally a respiratory sensor, wherein the physiological data comprise the speed and completion of the movements of one or both hands and/or arms, and the ratio of inspiration duration to expiration duration, respiratory frequency, respiratory period and/or tidal volume;
c. evaluating the physiological data;
d. assigning the physiological data to an acoustic part-signal or an acoustic signal series;
e. creating a series of sounds composed of at least two acoustic part-signals or signal series; and
f. outputting the series of sounds to the person,
wherein the combination and output of the acoustic signal series to form an overall acoustic sequence takes place according to a connective logic that follows the musical principles of European and/or non-European art music, selected from the series of keys and the degrees defined therein, series of melodic and harmonic progressions, series of musical form elements, series of timbres, rhythms and tempos, wherein these principles are present in a database, and the acoustic signal is formed into an acoustic pattern that develops anew and uniquely in its chronological profile each time.

## Revendications

1. Procédé destiné à délivrer un signal acoustique qui est composé de séquences de signaux acoustiques, elles-mêmes constituées de séquences acoustiques enchaînées d'une durée de 0,5 à 70 secondes et/ou de signaux partiels, la structure du signal acoustique se basant sur des données physiologiques, **caractérisé en ce que** ledit procédé comprend les étapes suivantes :
- fournir des données physiologiques sélectionnées d'une personne qui ont été saisies par capteur et qui comprennent au moins le rapport entre la durée d'inspiration et la durée d'expiration,
- discrétiser, quantifier et catégoriser l'évolution de paramètres des données physiologiques sélectionnées parmi les paramètres suivants : le rapport inspiration/expiration, la fréquence respiratoire, la période respiratoire, le volume respiratoire, les mouvements des doigts, des mains et des bras, ainsi que les tendances temporelles de ces paramètres, en attribuant, lors du processus de catégorisation, les signaux partiels et/ou les séquences de signaux à un rapport entre les paramètres physiologiques, à une enveloppe énergétique et à des segments musicaux,
- attribuer aux catégories des paramètres ainsi obtenues des séquences de signaux acoustiques en tenant compte d'au moins quatre parties musicales constituant des sous-sections structurantes d'une œuvre musicale, ainsi que d'une enveloppe énergétique,
- combiner en temps réel les séquences de signaux acoustiques conformément à l'évolution des paramètres catégorisés des données physiologiques pour former un signal acoustique final, puis délivrer ledit signal,
la combinaison et la délivrance des séquences de signaux acoustiques sous la forme d'une séquence acoustique complète s'effectuant selon une logique de liaison qui suit les lois musicales régissant la musique savante européenne et/ou non européenne et étant choisies parmi la succession de tonalités et des degrés qui y sont associés, la succession de progressions mélodiques et harmoniques, la succession de parties musicales, la succession de timbres, rythmes et tempos, ces lois étant contenues dans une base de données, et le signal acoustique étant transformé en une image sonore qui, au fil de son évolution dans le temps, renaît à chaque fois sous une forme nouvelle et unique, et
l'attribution des séquences de signaux acoustiques auxdites catégories s'effectuant selon une règle de formation qui veut qu'au moins quatre sections d'une période déterminée de 10 à 20 minutes soient remplies de séquences de signaux acoustiques corrélées à l'état physiologique respectif en tenant compte de l'administration de stimuli en vue d'un rapprochement à des valeurs cibles et de consigne à normes physiologiques variables ainsi qu'en tenant compte des règles musicales, ce qui permet d'obtenir une séquence acoustique globale continue et unique à chaque fois.

2. Procédé selon la revendication 1, **caractérisé en ce que** les paramètres utilisés dans le cas de données physiologiques à évolution périodique sont la durée de la période et/ou l'évolution de l'amplitude.

3. Procédé selon la revendication 1 ou 2, **caractérisé par** l'utilisation d'une unité de mémoire présentant une base de données contenant une multitude de signaux acoustiques partiels et/ou de séquences de signaux.

4. Procédé selon l'une quelconque des revendications 1 à 3, **caractérisé en ce que** les données physiologiques sélectionnées et saisies par capteur d'une personne comprennent en outre la fréquence respiratoire, la période respiratoire et/ou le volume respiratoire.

5. Procédé selon l'une quelconque des revendications 1 à 4, **caractérisé en ce que** les données physiologiques sélectionnées saisies par capteur sont fournies par un capteur respiratoire, une caméra, un capteur de mouvement ou un dispositif de contrôle gestuel infrarouge 3D, et qu'en option, un capteur de fréquence cardiaque peut être utilisé de manière supplémentaire.

6. Procédé selon l'une quelconque des revendications 1 à 5, **caractérisé en ce que** les données physiologiques sélectionnées saisies par capteur sont fournies par des dispositifs de ventilation mécanique et de monitorage de données patients.

7. Procédé selon l'une quelconque des revendications 1 à 6, **caractérisé en ce que** les signaux acoustiques partiels et/ou les séquences de signaux peuvent être relié(e)s en vue d'obtenir une multitude de séquences différentes formant le signal acoustique, les séquences présentant notamment une variation des séquences de signaux, de la densité de la succession tonale, de la hauteur tonale, du nombre de voix, du volume sonore, de l'articulation, du timbre, du tempo et/ou du rythme.

8. Procédé selon l'une quelconque des revendications 1 à 7, **caractérisé en ce que** les signaux acoustiques partiels et/ou les séquences de signaux peuvent être relié(e)s en vue d'obtenir une multitude de séquences différentes, et que les signaux acoustiques partiels et/ou les séquences de signaux présentent une variation de leurs attributs sonores, notamment du type de son, du timbre, de la hauteur tonale, de l'articulation, et/ou du volume sonore.

9. Procédé selon l'une quelconque des revendications 1 à 8, **caractérisé par** la fourniture de données physiologiques sélectionnées et saisies par capteur d'une personne, lesquelles comprennent des mouvements d'une main ou des deux mains et/ou d'un bras ou des deux bras, notamment la rapidité et le degré d'accomplissement desdits mouvements.

10. Dispositif adapté pour mettre en œuvre le procédé selon l'une quelconque des revendications 1 à 9, **caractérisé en ce que** le dispositif contient au moins les éléments suivants :
- au moins un capteur (2) destiné à saisir des données physiologiques sélectionnées d'une personne qui comprennent le rapport entre la durée d'inspiration et la durée d'expiration (I:E) et/ou les mouvements des mains,
- un module STAT (3) destiné à discrétiser, quantifier et catégoriser l'évolution de paramètres prédéfinis des données physiologiques,
- un sélecteur (8) destiné à attribuer des séquences de signaux acoustiques aux catégories et à la composition algorithmique conformément à l'évolution des paramètres catégorisés des données physiologiques, et
- un intégrateur (10) destiné à combiner en continu les séquences de signaux acoustiques en un signal acoustique et à délivrer le signal, les signaux acoustiques partiels et/ou les séquences de signaux étant stocké(e)s dans une base de données et réparti(e)s en au moins 4 catégories :
a. la catégorie 1 comprenant des signaux partiels et/ou des séquences de signaux qui correspondent à un rapport entre la durée d'inspiration et la durée d'expiration de 1:1 ;
b. la catégorie 2 comprenant des signaux partiels et/ou des séquences de signaux qui correspondent à un rapport entre la durée d'inspiration et la durée d'expiration de 1:2 ;
c. la catégorie 3 comprenant des signaux partiels et/ou des séquences de signaux qui correspondent à un rapport entre la durée d'inspiration et la durée d'expiration de 1:3 ;
d. la catégorie 4 comprenant des signaux partiels et/ou des séquences de signaux qui correspondent à des mouvements déterminés des doigts, des mains et des bras.

11. Dispositif selon la revendication 10, **caractérisé en ce que** les catégories 1 à 3 sont subdivisées dans les parties suivantes : "INTRO", "STIM1", "STIM2", "END" et que la catégorie 4 est attribuée à une ou plusieurs parties qui sont de préférence désignées en fonction des parties de corps concernées : doigts, mains et bras.

12. Dispositif selon la revendication 11, **caractérisé en ce que** les règles de la logique de liaison musicale utilisées pour les signaux acoustiques partiels et/ou les séquences de signaux ainsi que pour la succession des segments temporels individuels et l'enveloppe énergétique sont stockées dans une base de données.

13. Procédé mis en œuvre par ordinateur destiné à attribuer des données physiologiques à au moins un signal acoustique partiel ou à une séquence de signaux acoustiques, et à générer une séquence sonore constituée d'au moins deux signaux acoustiques conformément à un procédé selon les revendications 3 à 9, ledit procédé comprenant les étapes suivantes :
a. fournir une base de données comprenant une multitude de signaux acoustiques partiels et/ou de séquences de signaux ;
b. déterminer des données physiologiques au moins à l'aide d'un capteur respiratoire ou d'un capteur de mouvement, les données physiologiques comprenant au moins le rapport entre la durée d'inspiration et la durée d'expiration ;
c. évaluer les données physiologiques ;
d. attribuer les données physiologiques à un signal acoustique partiel ou à une séquence de signaux acoustiques ;
e. attribuer, de manière complémentaire, les signaux acoustiques partiels et/ou les séquences de signaux à au moins quatre parties musicales et à une enveloppe énergétique ; et
f. générer et délivrer une séquence sonore constituée d'au moins deux signaux acoustiques partiels, séquences de signaux ou signaux.

14. Utilisation du procédé selon l'une quelconque des revendications 1 à 9, adapté pour moduler la fréquence respiratoire, la période respiratoire et/ou le rapport entre la durée d'inspiration et la durée d'expiration ou pour entraîner la coordination des mouvements des mains.

15. Procédé selon l'une quelconque des revendications 1 à 9, adapté pour moduler le schéma respiratoire d'une personne, ledit procédé comprenant les étapes suivantes :
a. fournir une base de données comprenant une multitude de signaux acoustiques partiels et/ou de séquences de signaux ;
b. déterminer des données physiologiques d'une personne à l'aide d'au moins un capteur respiratoire et de préférence d'un capteur de mouvement, les données physiologiques comprenant au moins le rapport entre la durée d'inspiration et la durée d'expiration, et, en option, la fréquence respiratoire, la période respiratoire et/ou le volume respiratoire et des mouvements spécifiques des doigts, des mains ou des bras ainsi que la vitesse et le degré d'accomplissement avec lesquels sont effectués les mouvements d'une main ou des deux mains et/ou d'un bras ou des deux bras.
c. évaluer les données physiologiques ;
d. attribuer les données physiologiques à un signal acoustique partiel ou à une séquence de signaux acoustiques ;
e. attribuer les signaux acoustiques partiels et/ou les séquences de signaux à au moins quatre parties musicales et à une enveloppe énergétique ;
f. générer une séquence sonore constituée d'au moins deux signaux acoustiques partiels ou séquences de signaux ; et
g. délivrer la séquence sonore à la personne,
la combinaison et la délivrance des séquences de signaux acoustiques sous la forme d'une séquence acoustique complète s'effectuant selon une logique de liaison qui suit les lois musicales régissant la musique savante européenne et/ou non européenne et étant choisies parmi la succession de tonalités et les degrés qui y sont associés, la succession de progressions mélodiques et harmoniques, la succession de parties musicales, la succession de timbres, rythmes et tempos, ces lois étant contenues dans une base de données, et le signal acoustique étant transformé en une image sonore qui, au fil de son évolution dans le temps, renaît à chaque fois sous une forme nouvelle et unique.

16. Procédé selon la revendication 14, dans lequel la modulation du schéma respiratoire comprend la prolongation de l'expiration par rapport à l'inspiration.

17. Procédé destiné à l'entraînement de la coordination des mains d'une personne selon l'un quelconque des procédés 1 à 9, ledit procédé comprenant les étapes suivantes :
a. fournir une base de données comprenant une multitude de signaux acoustiques partiels et/ou de séquences de signaux ;
b. fournir des données physiologiques de la personne qui ont été saisies par un capteur de mouvement et, en option, par un capteur respiratoire, les données physiologiques comprenant la vitesse et le degré d'accomplissement des mouvements d'une main ou des deux mains et/ou d'un bras ou des deux bras, et le rapport entre la durée d'inspiration et la durée d'expiration, la fréquence respiratoire, la période respiratoire et/ou le volume respiratoire ;
c. évaluer les données physiologiques ;
d. attribuer les données physiologiques à un signal acoustique partiel ou à une séquence de signaux acoustiques ;
e. générer une séquence sonore constituée d'au moins deux signaux acoustiques partiels ou séquences de signaux ; et
f. délivrer la séquence sonore à la personne,
la combinaison et la délivrance des séquences de signaux acoustiques sous la forme d'une séquence acoustique complète s'effectuant selon une logique de liaison qui suit les lois musicales régissant la musique savante européenne et/ou non européenne et étant choisies parmi la succession de tonalités et les degrés qui y sont associés, la succession de progressions mélodiques et harmoniques, la succession de parties musicales, la succession de timbres, rythmes et tempos, ces lois étant contenues dans une base de données, et le signal acoustique étant transformé en une image sonore qui, au fil de son évolution dans le temps, renaît à chaque fois sous une forme nouvelle et unique.
